(19) Europäisches Patentamt
European Patent Office
Office européen des brevets

(11) **EP 4 549 602 A1**

(12) **EUROPEAN PATENT APPLICATION**

(43) Date of publication:
07.05.2025 Bulletin 2025/19

(21) Application number: 24210602.9

(22) Date of filing: 04.11.2024

(51) International Patent Classification (IPC):
$C22B\ 3/20^{(2006.01)}$     $C22B\ 26/12^{(2006.01)}$

(52) Cooperative Patent Classification (CPC):
C22B 3/20; C22B 26/12; G01N 33/1813

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 03.11.2023 US 202363595784 P

(71) Applicants:
• Services Pétroliers Schlumberger
75007 Paris (FR)
Designated Contracting States:
FR
• Schlumberger Technology B.V.
2514 JG The Hague (NL)
Designated Contracting States:
AL AT BE BG CH CY CZ DE DK EE ES FI GB GR
HR HU IE IS IT LI LT LU LV MC ME MK MT NL NO
PL PT RO RS SE SI SK SM TR

(72) Inventors:
• Madani Sani, Fazlollah
Sugar Land, 77478 (US)
• Dubey, Sharad
Sugar Land, 77478 (US)
• Friberg, Lothar
Sugar Land, 77478 (US)
• Biswal, Prayag
Houston, 77056-272 (US)
• Dsouza, Rithika
411006 Pune (IN)
• Nirgudkar, Prasanna
Sugar Land, 77478 (US)

(74) Representative: Schlumberger Intellectual
Property Department
Parkstraat 83
2514 JG Den Haag (NL)

(54) **QUANTITATIVE BRINE ANALYSIS FOR MINERAL EXTRACTION**

(57) A method according to the present invention includes obtaining parameters of a sample brine extracted from the aqueous source; determining a brine pretreatment score based on one or more of the parameters of the sample brine; determining a brine extraction score based on one or more of the parameters of the sample brine; determining a brine post-extraction score based on one or more of the parameters of the sample brine; determining a brine total score based on the brine pretreatment score, the brine extraction score, and the brine post-extraction score; and outputting a visualization of at least one of: the brine pretreatment score, the brine extraction score, the brine post-extraction score, or the brine total score to a display.

EP 4 549 602 A1

A WORKFLOW FOR QUANTITATIVE BRINE ANALYSIS

IDENTIFY BRINE COMPOSITION — 505

DETERMINE A BRINE PRE-TREATMENT SCORE — 510

DETERMINE A BRINE EXTRACTION SCORE — 515

DETERMINE A BRINE POST EXTRACTION SCORE — 520

DETERMINE A BRINE TOTAL SCORE — 525

SELECT A BRINE FOR PERFORMING A MINERAL EXTRACTION PROCESS BASED ON THE SCORES — 530

SELECT ONE OR MORE BRINE PRE-TREATMENTS FOR THE MINERAL EXTRACTION PROCESS BASED ON THE BRINE PRE-TREATMENT SCORE — 535

SELECT ONE OR MORE BRINE POST-PROCESSING TREATMENTS FOR THE MINERAL EXTRACTION PROCESS BASED ON THE BRINE POST-PROCESSING SCORE — 540

PERFORM MINERAL EXTRACTION ON THE BRINE — 545

*FIG. 5* — 500

## Description

## Cross-Reference to Related Applications

The present Application for Patent claims priority to U.S. Provisional Application No. 63/595,784, filed November 3, 2023, which is hereby expressly incorporated by reference herein in its entirety.

## BACKGROUND

### Field of the Disclosure

Aspects of the present disclosure relate to mineral recovery from aqueous sources and, more particularly, to techniques for quantitative analysis of brines for mineral extraction.

### Description of Related Art

As the energy industry shifts away from dependence on fossil fuels and towards more sustainable and eco-friendly sources of energy, the production of critical minerals has become increasingly crucial. For example, some of these minerals are essential components of energy storage units, making their availability a key factor in the widespread adoption of renewable energy technologies. Lithium holds a significant position among critical minerals, primarily because of its essential role in powering batteries for electric vehicles, portable electronics, and renewable energy storage systems. For example, various electrical storage devices, such as batteries, supercapacitors, and similar devices commonly use lithium to mediate the storage and release of chemical potential energy as electrical current. Other critical minerals may be used in batteries and/or other devices related to renewables energy for example. As the demand for renewable, but non-transportable, energy sources such as solar and wind energy grows, so does the demand for technologies to store energy generated from such sources and, in turn, the demand for lithium also grows.

There is growing interest in exploring unconventional sources of critical minerals, as they offer sustainable alternatives to traditional mining sources. Unconventional sources aqueous solutions (or brines), which may include natural brines from salars, continental brines, petro-brines, clay brines, surface brines, seawater, lake water, etc. Critical minerals such as lithium, manganese, nickel, cobalt can be extracted using direct aqueous extraction. Additionally, side streams from various industries such as produced waters, geothermal brines, mine tailings, and acid mine drainage can also serve as potential sources of these minerals. These sources present promising opportunities to supplement traditional mining sources and reduce the environmental impact of mineral extraction.

Aqueous solutions subjected to extraction can have diverse compositions of critical minerals as well as different contaminants. The mining industry has numerous techniques for the extraction of critical minerals. Hard rock mining with acid digestion is common, but labor and energy intensive.

Extraction of critical minerals from brines can be performed using other techniques such as solvent extraction and evaporation ponds. However, these methods typically have low yields and negative environmental impacts. One prevalent technique involves utilizing surface waters and salar lakes. This method comprises pumping brine into evaporation ponds with the addition of chemical agents to selectively precipitate critical mineral substances, the brine is evaporated (e.g., by solar energy) leaving behind concentrated critical mineral salts, such as lithium, magnesium or boron salts. Extraction by evaporation techniques may be slow, environmentally disruptive (e.g., due to water usage, land disruptions, and potential leakage of toxic chemicals into surrounding areas), and dependent on climate conditions. For example, lithium extraction with evaporation ponds may take months (e.g., up to 18 months) to complete, recovering roughly 50-60% of the original lithium.

Recently, sorbent and ion exchange (IX) methods have gained attention, especially for lithium extraction, due to their efficiency and environmentally-friendly characteristics. For example, direct lithium extraction (DLE) by sorbents extracts lithium directly from brines without the need for evaporation ponds, reducing water usage and minimizing the impact on surrounding ecosystems.

Direct extraction of critical minerals, such as DLE, is a set of advanced technologies designed to extract critical minerals from brine sources, such as salt flats or underground aquifers. In this application, a brine source or brine is defined as an aqueous source containing one or more dissolved elements of interest, including ions derived from the element of interest. Such brine may originate from natural or artificial source and can include tailings, wastewater, battery recycling, oilfield stream, seawater, hard rock leachate, etc. Rich brines may be found for instance in regions with high geological activity, such as salt flats (salars) and underground aquifers. The element of interest may include more specifically one or more of lithium, nickel, cobalt, manganese, magnesium, potassium, copper, iron, zinc, aluminum, molybdenum, vanadium, gallium, rubidium, strontium, boron, scandium, bromine, etc.

Direct extraction aims to accelerate the extraction process while avoiding the need for extensive evaporation and energy consumption along with minimizing environmental impacts.

Direct extraction offers several benefits over traditional methods. Direct extraction boasts lower energy consumption compared to hard rock mining. Direct extraction methods can significantly reduce the time required to extract the element of interest from brine sources, potentially down to a matter of hours. Direct extraction technologies can be designed to require less space compared to expansive evaporation ponds. Direct extraction technologies have the potential to reduce water usage, a critical consideration in water-scarce regions. By reducing the need for large evaporation ponds, less chemical and acid consumptions, and lowering the risk of chemical leakage, direct extraction methods can have a reduced environmental impact. Direct extraction technologies can be easily scaled up or down based on demand, unlike traditional methods, which rely heavily on type of mineral deposit, its grade, accessibility, and climate conditions.

While direct extraction methods offer promising advantages, researchers and engineers are still working to optimize the efficiency, selectivity, and cost-effectiveness of these technologies. Further, mineral extraction from brine is sensitive to the composition of feed brines, specifically, to the presence of dissolved or dispersed impurities. Moreover, the extraction process may need to meet certain limits of minerals of interest and impurity concentrations.

Accordingly, techniques are needed for the analysis of the quality of brines before extraction, such as to determine if any pretreatment is required and what the expected yield will be for the extraction process.

## SUMMARY

One aspect provides a method of quantitative brine analysis. The method may include obtaining parameters of a sample brine extracted from the aqueous source; determining a brine pretreatment score based on one or more of the parameters of the sample brine. The method may include determining a brine extraction score based on one or more of the parameters of the sample brine. The method may include determining a brine post-extraction score based on one or more of the parameters of the sample brine. The method may include determining a brine total score based on the brine pretreatment score, the brine extraction score, and the brine post-extraction score. The method may include outputting a visualization of at least one of: the brine pretreatment score, the brine extraction score, the brine post-extraction score, or the brine total score to a display.

Another aspect provides a non-transitory computer readable medium. The non-transitory computer-readable medium stores computer-executable code. The computer-executable code may include code for obtaining parameters of a sample brine extracted from the aqueous source. The computer-executable code may include code for determining a brine pretreatment score based on one or more of the parameters of the sample brine. The computer-executable code may include code for determining a brine extraction score based on one or more of the parameters of the sample brine. The computer-executable code may include code for determining a brine post-extraction score based on one or more of the parameters of the sample brine. The computer-executable code may include code for determining a brine total score based on the brine pretreatment score, the brine extraction score, and the brine post-extraction score. The computer-executable code may include code for outputting a visualization of at least one of: the brine pretreatment score, the brine extraction score, the brine post-extraction score, or the brine total score to a display.

The following description and the appended figures set forth certain features for purposes of illustration.

## BRIEF DESCRIPTION OF DRAWINGS

The appended figures depict certain features of the various aspects described herein and are not to be considered limiting the scope of this disclosure.

**Figure 1** is a process diagram of a recovery process according to certain aspects.

**Figure 2** is a process diagram of another recovery process according to certain aspects.

**Figures 3A-3D** illustrate a process of brine pretreatment stages according to certain aspects.

**Figure 4** depicts a process flow for direct recovery with brine pre-treatment according to certain aspects.

**Figure 5** illustrates an example workflow for quantitative brine analysis.

**Figures 6A-6B** depict a table illustrating example input to a system for quantitative brine analysis.

**Figure 7A** illustrates an example brine extraction subscore for lithium concentration.

**Figure 7B** illustrates an example brine extraction subscore for temperature.

**Figure 8** illustrates example visualizations of quantitative brine assessment scores.

**Figure 9** illustrates example pre-treatment subscores stored in a database.

**Figure 10** illustrates example extraction subscores stored in a database.

**Figure 11** illustrates example post processing subscores stored in a database.

**Figure 12** illustrates example total scores stored in a database.

**Figure 13** illustrates an example system for quantitative brine analysis

**Figure 14** illustrates example code for brine assessment, brine assessment visualization, and assessing a brine database.

**DETAILED DESCRIPTION**

Aspects of the present disclosure provide apparatuses, methods, systems, and computer-readable mediums for quantitative brine analysis for mineral extraction. In some examples below, the techniques have been applied to lithium extraction but similar techniques may be applied to brine for extraction or recovery of other elements of interest.

In some aspects, a brine assessment workflow is provided, which may be useful in quickly identifying the quality of brines. The quantitative analysis of the brine may be used to assess whether the brine meets composition requirements for the mineral extraction processes as well as the expected mineral extraction yield. This analysis may aid in selecting the optimal extraction processes, pre-treatments, and/or technologies for the mineral extraction to maximize the lithium recovery and system reliability, while minimizing processing time and costs and, thereby, profitability. In some aspects, approaches are provided for brine assessment visualization.

*Introduction to Mineral Extraction*

Elements of interest such as lithium can be extracted from aqueous sources such as salt flats (salars), salt lakes, surface brines, continental brines, seawater, petro-lithium brine, mining brines (e.g., as byproducts of extracting lithium-containing shale or mica, or spodumene), geothermal brines, battery recycling effluent, and other aqueous sources. Minerals, such as lithium-bearing clays like hectorite and magnesium-rich minerals can also be effectively dispersed in water for direct extraction processes. Moreover, direct extraction methods offer the potential for recovering radioactive isotopes like strontium and caesium from nuclear wastewater sources, as well as extracting rare earth metals from mine leach solutions. The processes described herein can be used to extract lithium from such sources.

Direct extraction flowsheet may involve the actual element of interest extraction stage, using for instance ion withdrawal or electrochemical processes, and may include additional post-extraction stages to enhance quality of the extracted element of interest (for instance, for lithium, to obtain battery-grade lithium quality). Such stages may include impurity removal (e.g., coagulation, flocculation, ion exchange, or chemical treatment), concentration (e.g., using membrane separation or evaporation), and conversion (e.g., for lithium, from lithium chloride to lithium hydroxide and/or carbonate).

In some examples, ion withdrawal processes involve passing the brine through a withdrawal material, for instance a resin, that has a strong affinity for ions derived from the element of interest, such as lithium ions. The resin selectively captures the desired ions from the brine as it flows through, while allowing other ions to remain in the brine. Subsequent processes release the captured ions for further refinement. In some examples, membranes with specific pore sizes allow desired ions to pass through while blocking other ions. Pressure-driven or electrically driven systems push the brine through these membranes, effectively separating the element of interest, such as lithium from the other components. In some examples, an electric field is used to selectively move the desired ions through ion-selective membranes. For example, electrodialysis uses voltage gradients to drive the desired ions to migrate, resulting in a concentrated stream. In some examples, organic solvents can be used to extract the ions derived from the element of interest from the brine. The solvent contains chemicals to which desired ions bind strongly, allowing them to be separated from the rest of the brine. The solvent is then stripped of the desired ions, leaving behind concentrated compounds.

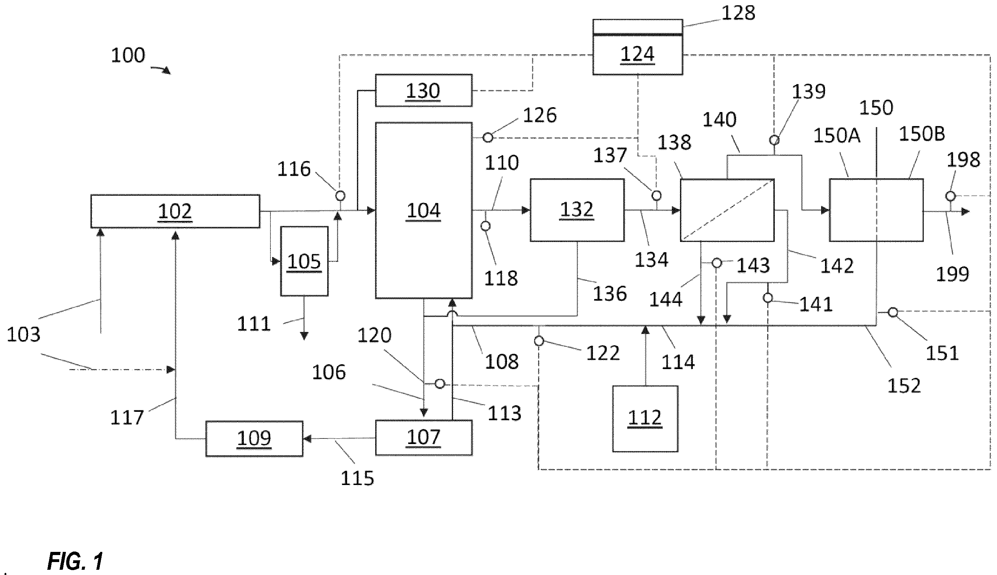

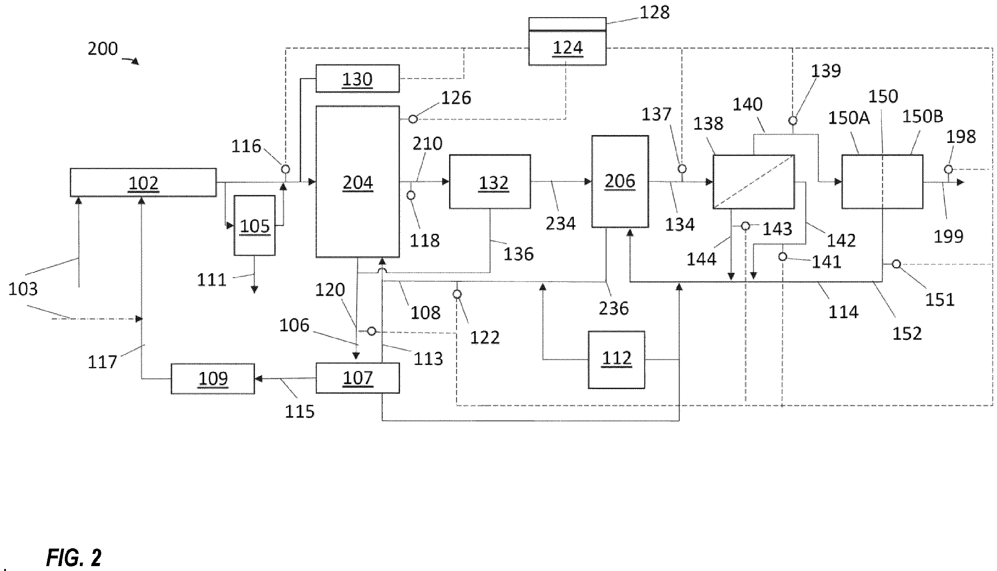

The following **Figure 1** and **Figure 2** show an example DLE process. Such process could be applied to extract other elements of interest. The direct extraction process as per the disclosure is not limited to the following process.

**Figure 1** is a process diagram depicting stages of an example lithium recovery process 100 according to one embodiment. The lithium recovery process 100 of **Figure 1** generally uses a sorption/desorption process to extract lithium from a brine along with a membrane concentration process to increase solution concentration of lithium for downstream conversions to lithium carbonate and/or lithium hydroxide hydrate. The sorption portion of the process can be either absorption or adsorption, or a mixture thereof, depending on the medium used to accomplish the sorption.

Lithium-containing brine from a brine source 102 is brought to the inlet of a lithium extractor 104. The lithium extractor 104 is a sorption unit, with a sorption medium that selectively absorbs lithium. In the lithium extractor 104, a resin is disposed within a vessel to provide exposure of the resin to a lithium-containing brine. The resin may be stationary or fluidized within the vessel, or the resin may be conveyed through one or more vessels or zones for contacting with the brine, for example in a counter-current format (counter-current sorption/desorption). The resin adsorbs lithium from the brine source 102 leaving a lithium-depleted brine 106, which exits the lithium extractor 104. Sorption may be encouraged by heating the brine source 102 to a temperature, for instance of at least 70°F, where the brine source 102 has a natural temperature that is below that range. Water may be separated from the lithium-depleted brine 106 using a water recovery process 107, which can be a thermal process, filtration process, or membrane process. The water recovery process recovers a water stream 113 from the lithium-depleted brine 106, yielding an impurity stream 115. The water stream 113 is re-used in the lithium extractor 104, as further described below, and the impurity stream 115 is routed to a purification process 109, which can be any suitable purification process such as a rapid infiltration process, or other filtration or membrane process, or combination of processes, before a clean brine 117 is returned to the brine source.

Lithium recovery processing can be enhanced by lowering pH in the brine source 102. A lithium-selective additive sweep 103 can be applied directly to the brine source 102 to preferentially encourage lithium to migrate toward the feed of the lithium extractor 104. For example, where the brine source contains lithium chloride, a sweep 103 of hydrochloric acid (HCl) can be injected in the brine source 102 at a location remote from the feed to the lithium extractor 104 to enhance lithium recovery processing. Applying an acid sweep such that pH of the feed to the lithium extractor 104 is from 5.5 to 7.0, for example about 6.0, can, for example, mobilize lithium from mineral deposits in and around the brine source. Low pH in the brine source 102 can be remediated after exhaustion of the lithium, if desired, by adding a suitable basic compound, such as sodium or potassium hydroxide to raise pH to its original value. In one alternate method, the lithium-selective additive sweep 103 can be added to brine returned to the brine source 102 from the lithium recovery process 100, so the returned brine can be used as a vehicle to deliver the sweep 103. Other materials that can be used as lithium-selective additive sweep include lithium-selective absorbents, polymers, dissolved gases, liquid ionexchange fluids, and other materials. Selection of such materials depend on geometry and composition of the brine source 102 and location of brine withdrawal from, and injection into, the brine source 102.

An optional pretreatment process may be performed on the brine from the brine source 102 to reduce impurities that might impact the performance of the lithium extractor 104. For example, a compatible reagent can be added to the lithium-containing brine from the brine source 102 in a pretreat unit 105 to reduce impurities such as iron ions and other metallic ions prior to exposing the lithium extractor 104 to the brine. The impurities can be removed as an impurity stream 111. Such treatments can be helpful where impurity levels are too high to treat directly at the brine source 102. In another version of a pretreatment process, which can be combined with the pretreatment process described above, an impurity absorber or filter, or both, can be used to trap impurities that might affect the performance of the lithium extractor 104. A medium selective to such impurities, such as an ion exchange medium or a filtration medium, can preferentially remove impurities such as silica and divalent ions that can degrade the lithium sorption capacity of the lithium extractor 104.

In the lithium extractor 104, resin loaded with lithium is contacted with an eluent stream 108 that removes lithium from the loaded resin. A lithium extract stream 110 exits the lithium extractor 104. Most non-lithium ions in the original brine stream exit with the lithium-depleted brine, so the lithium extract stream 110 has, at most, low levels of impurities. The eluent stream 108 may be deionized water, or water with low levels of lithium or other harmless ions to the process. A water source 112, such as a deionized water source or a water purifier, may be used to provide water for the eluent stream 108. Here, a lithium-containing stream 114 is recycled from downstream operations to provide water for the eluent stream 108. The water stream 113 is also used to provide water for the eluent stream 108. Water can be added from the water source 112 where make-up water is needed to reach a target solution concentration in the lithium extract stream 110, or to lower solution concentration of ions, such as lithium or other ions, to a target level in the eluent stream 108. Using recycled streams for the eluent stream 108 reduces, and can eliminate, the need for fresh water sources. Lithium desorption can be enhanced by heating the eluent stream to certain temperatures, for instance a temperature above 140°F.

Composition analytical instruments can be coupled to any of the incoming and outgoing streams of the lithium extractor 104 to provide data for controlling and optimizing the operation of the lithium extractor 104. The composition analytical instruments may be any or all of pH probes, ion-selective electrodes, conductivity instruments, permittivity instruments, specific gravity instruments, turbidity instruments, electrochemical instruments (e.g., ionophoric electrodes and membranes), chromatographs or other differential separation instruments, spectrometers (Fourier-transform infrared (FTIR), nuclear magnetic resonance (NMR), flame ionization or emission, mass spectrometers, X-ray fluorescence, etc.) or other optical instruments, and the like. One or more instruments can be used for each, or any stream, and multiple instruments based on different technologies can be used to reduce measurement uncertainty for any stream. Temperature and pressure gauges along with analytical tools for fluid physical properties can also be coupled to any desired stream.

Thus, one or more composition analytical instruments 116 may be disposed at the brine source 102 or at the brine inlet to the lithium extractor 104, one or more lithium extract composition analytical instruments 118 may be disposed in the lithium extract stream 110, one or more lithium-depleted stream composition analytical instruments 120 may be disposed in the lithium-depleted brine 106, and one or more eluent composition analytical instruments 122 may be disposed in the eluent stream 108. Each, or any, of the composition analytical instruments 116, 118, 120, and 122 may also sense other conditions of each respective stream, such as temperature, pressure, and fluid physical properties. Each of the instruments 116, 118, 120, and 122, if used, may be operatively coupled to a controller 124 configured to receive signals from each of the instruments 116, 118, 120, and 122 representing composition, and optionally other conditions, of the corresponding streams. Other instruments can optionally be used to sense other conditions of the respective streams, separately, and provide signals representing those other conditions to the controller 124. The controller 124 can be configured to adjust process conditions of the lithium extractor 104 based on the signals from the instruments 116, 118, 120, and 122, and any other instruments that might be applied. For example, the controller 124 can adjust flow rate of the brine source 102, flow rate of the eluent stream 108, flow rate of water from the water source 112, and/or flow rate of the lithium-containing stream 114 based on the signals. The controller 124 can also be configured to monitor lithium uptake of the absorbent, for example based on lithium detected in the brine source 102 by the brine source instrument(s) 116 and lithium detected in the lithium-depleted brine 106. The controller 124 can be configured to adjust resin loading time (e.g., time spent loading the resin with lithium), resin cycle time (resin loading time plus resin unloading time), eluent residence time, or other process parameters based on lithium uptake. Lithium unloading can also be similarly monitored, and process adjustments made by the controller 124.

Other instruments can be used with the lithium extractor 104. For example, imaging or "signature" instruments of various types, such as NMR and X-ray power diffraction (XRD) instruments, can be used and operatively coupled to the controller 124. Thus, a signature instrument 126 can be coupled to the lithium extractor 104 to generate a signal representing the effect of lithium extractor 104 on an electric field, magnetic field, or propagating electromagnetic radiation. The signal can be thought of as a signature representative of the process conditions. A simulator or machine learning system can be used to process the signals from any or all of the instruments 116, 118, 120, 122, and 126 and output process set points such as flow rates, temperatures, and the like. For example, an advisory system 128 may be operatively coupled to the controller 124 and to the instruments 116, 118, 120, 122, and 126 to compute process targets or ranges for implementation by the controller 124 or to output process recommendations to the operators. The signature instrument 126, for example, may be able to highlight more esoteric process conditions such as channeling, plugging, or scaling in the resin and to signal an operator that such process conditions are occurring. The signature instrument 126, for example, may inject some form of electromagnetic radiation into the lithium extractor 104 itself. The radiation interacts with the interior of the lithium extractor 104, and the resulting radiation "signature" is detected to derive information about the interior of the extractor 104.

Another example of an instrument system that can be used to track operation and performance of the lithium extractor 104 is a physical replica of the extractor.

An absorber-analyzer 130 can be coupled to the brine source 102 to serve as a test unit to monitor for changing composition of the brine source 102. The absorber-analyzer 130 is a small sorption unit loaded with the same medium used for lithium separation in the lithium extractor 104. The absorber-analyzer 130 can be used for detecting and monitoring impurity levels that can affect the performance of the sorption medium in the lithium extractor 104. A slipstream of the brine source 102 can be routed to the absorber-analyzer 130, and instruments, such as pH probes, conductivity sensors, temperature and pressure gauges, and composition analyzer can be applied to monitor changing conditions within the absorber-analyzer 130. This data then can be used to predict changing conditions of the lithium extractor 104. The instruments, encompassing any or all of those mentioned above, can be operatively coupled to the controller 124. The controller 124 can monitor the instruments of the absorber-analyzer 130 and apply predictive methods, such as simulators and machine learning systems, to control the lithium extractor 104 based on the readings from the absorber-analyzer instruments. Similarly, the instruments coupled to the lithium extractor 104 can also contribute to this purpose. Additionally, the instruments of the lithium extractor 104 and the absorber-analyzer 130 can be provided to the advisory system 128 to improve its output accuracy.

Another instrument system that can be used to track operation and performance of the lithium extractor 104 is a tracer detector. An easily detectible species with behavior toward the absorbent medium of the lithium extractor 104 is injected into the feed to the extractor 104 as a tracer, and detection of the tracer is applied to one or both of the streams 108 and 110 to monitor uptake of the tracer by the absorbent medium. The same tracer detector system can be applied to the absorber-analyzer, if desired. It should be noted that additional sample streams can be obtained from the lithium extractor 104 and/or the absorber-analyzer 130 to monitor conditions of the absorbent medium at intermediate locations between feed and effluent. The tracer detector system can provide analysis of changing conditions throughout the lithium extractor 104 during processing to control the extractor and diagnose intervention situations.

The advisory system 128 is a digital processing system that generally takes input data from the controller 124, and potentially directly from the instruments 116, 118, 120, 122, and 126, and those of the absorber-analyzer 130, and provides output to the controller 124. The output may be set points of various instruments of the process 100 or operating parameter targets that the controller 124 translates into set points for the instruments. The advisory system 128 may be co-located with the controller 124, or may be remote from the controller 124. The advisory system 128 may have direct data linkage to the controller 124, or may communicate with the controller 124 via a digital network. The advisory system 128 may be coupled to controllers of multiple lithium recovery processes like the process 100 to increase data available for the models used by the advisory system 128. In such cases, other digital processing systems, such as data aggregators, disaggregators, routers, and the like, may mediate communication between the advisory system 128 and the controllers 124. The advisory system 128 may also support data visualization and user reporting.

The lithium concentration in the lithium extract stream 110 may be restricted solely by solubility limit of the extracted lithium salt. Flow rate of the eluent stream 108 can be controlled to maximize lithium solution concentration in the extract stream 110. The lithium extractor 104 can boost lithium solution concentration, in some cases, by a factor of 20 or more. That is to say, a gain ratio of lithium solution concentration in the lithium extract stream 110 to lithium solution concentration in the brine source 102 can be a factor of 20 or more. Depending on lithium solution concentration of the brine source 102, the ratio can be almost arbitrarily large. Dilute brine sources will take time to load the absorbent medium in the extractor 104, but once loaded, the lithium can be unloaded at near the solubility limit in the extract stream 110.

The lithium extract stream 110 is routed to an impurity removal unit 132, which can be a filtration unit, ion exchange unit, membrane unit, flocculation unit, precipitation unit, electrochemical coagulation unit, density separation unit, or another chemical or physical treatment unit for removing non-lithium impurities such as silica, divalent metal ions and other particulates, which are known in the art. The impurity removal unit 132 produces a clean lithium extract stream 134, and may produce one or more impurity streams 136 that can be routed to the water recovery process 107, or to another advantageous use. Lithium solution concentration in the clean lithium extract stream 134 may be the same as that of the lithium extract stream 110, or may be less if chemical additives are used to remove impurities. If downstream concentrators are used to recover lithium, minimizing dilution during impurity removal can minimize concentrator duty.

A composition instrument 137, optionally also including temperature, pressure, and fluid physical properties instruments, can be coupled to the clean lithium extract stream 134, for example to monitor impurities that might pass through the impurity removal unit 132. Temperature and pressure of the clean lithium extract stream 134 can optionally be sensed separately. The composition instrument 137, and any other instruments optionally coupled to the clean lithium extract stream 134, can be operatively connected to the controller 124, which can be configured to control operation of the impurity removal unit 132 based on signals from the composition instrument 137 to target or minimize the levels of one or more impurities, such as silica and divalent ions.

The controller 124 can be further configured to operate the impurity removal unit 132, for instance based on signals from the composition instrument 137, to intensify removal of impurities by adjusting temperature, pressure, or sweep to increase separation of impurities.

The clean lithium extract stream 134 is routed to a concentrator 138. The concentrator 138 is a water removal process that produces a lithium concentrate stream 140 and a water stream 142. Here, the concentrator 138 is depicted as a membrane separator, but the concentrator 138 could also be an evaporator, such as a thermal evaporator, a force circulation evaporator (e.g., an evaporator that utilizes humidity of a gas), or a multi-effect evaporator, in some embodiments. The concentrator 138 may also produce a sweep effluent 144 that can be combined with other streams in a controlled fashion to target a salinity level in the eluent stream 108 and/or the concentrate stream. The water removal process of the concentrator 138 may use multiple membrane separation units (e.g., a membrane separation unit having a retentate side where the stream 134 flows and a permeate side separated from the retentate side by a membrane allowing ions but not lithium ion to flow through) and/or multiple evaporators in series and/or parallel. For the concentrator 138 with one or multiple membrane separation units, the lithium extract stream 134 is brought to a target pressure, for example using a pump of any convenient type. In an embodiment, the concentrator 138 uses a reverse osmosis system. In an embodiment, a stream may flow, for instance counter-current, on the permeate side of at least one of the membrane separation units. In an embodiment the concentrator 138 includes a counter-flow reverse osmosis system. A portion, or all, of any of the effluent streams of the concentrator 138, including the lithium concentrate stream 140 and the water stream 142, can be recycled to the lithium extractor 104 as the lithium-containing stream 114, or a component thereof, to be used as part of the eluent stream 108. Salinity in the eluent stream 108 can be controlled by mixing various salt-containing streams, with varying salinities, to meet a target. The concentrator 138 preferentially increases lithium solution concentration by a factor of at least about 20, in some cases, by removing water from the clean lithium extract stream 134. The permeate stream 142 is a fresh water stream that can be recycled to the lithium extractor 104 through the eluent stream 108.

Effluent streams of the concentrator 138 can have instruments operatively coupled to the controller 124. A composition instrument 139 can be coupled to the lithium concentrate stream 140. A composition instrument 141 can be coupled to the water stream 142. Where a sweep is used for the concentrator 138 with one or multiple membrane separation units, a composition instrument 143 can be coupled to the sweep effluent 144. Each of the instruments 139, 141, and 143 can optionally also include temperature, pressure, and fluid physical properties instruments, or such instruments can be separately coupled to the respective streams. Each of the instruments 139, 141, and 143, if used, can be operatively coupled to the controller 124, which can be configured to adjust operation of the concentrator 138 based on signals from the instruments 139, 141, and 143, and to adjust recycle of streams to the lithium extractor 104 based on the composition signals and/or the condition (pressure, temperature, etc.) signals from the instruments 139, 141, and 143. If lithium penetration occurs in the concentrator 138, the lithium can be recycled in the eluent stream 108 and recovered in the lithium extractor 104. If increased lithium concentration in the eluent stream 108 is detected due to lithium penetration in the lithium concentrator 138, the controller 124 can use more make-up water from the water source 112, or more or less of the recycled downstream streams from the concentrator 138 (e.g., the permeate stream 142) or the conversion process 150 (e.g., the water streams 152).

The lithium concentrate stream 140 is converted to a lithium hydroxide product 199, typically but not necessarily a lithium hydroxide monohydrate, in a conversion process 150. The conversion process 150 involves a first treatment 150A using sodium carbonate to convert lithium chloride to lithium carbonate followed by a second treatment 150B using calcium hydroxide to convert lithium carbonate to lithium hydroxide powder or hydrate paste. Either or both treatments may include evaporation, which encourages precipitation, but can also precipitate some impurities in the first treatment 150A. Either or both treatments may include physical separation, for example filtration or centrifugation to enhance the efficiency of the conversion process 150. A wash step can be performed on the filtrate to remove impurities with little loss of lithium. The wash effluent can be returned to the brine source 102. In alternate methods, direct conversion to lithium hydroxide may be accomplished using electrochemical methods.

The evaporation can produce one or more water streams 152 that can be used for recycling, for example to the eluent stream 108, potentially along with other streams that can be recycled to the lithium extractor 104 to target salinity concentration in the eluent stream 108. Where the water stream 142 of the concentrator 138 is the first water stream, one or more second water streams 152 are produced by the conversion process 150. Examples of other streams from the conversion process 150 that can be recycled include a portion of the lithium carbonate stream from the first treatment 150A and/or a portion of the lithium hydroxide from the second treatment 150B, with or without other streams from the conversion process 150 or the concentrator 138, to the eluent stream 108. All the clean lithium-containing streams derived from the lithium-containing stream 114 can be manifolded to the eluent stream 108, with flow controls operatively coupled to the controller 124, which can be configured to adjust flow rates of the various streams, along with make-up water from the water source 112, or water recovered in the water recovery process 107, to optimize composition of the eluent stream 108 for lithium unloading from the absorbent medium. As described above, composition of the various streams can be sensed, and signals routed to the controller 124 to determine an eluent stream composition for optimal unloading rate and lithium solution concentration. A composition instrument 151 can be coupled to the water streams 152 to monitor for impurities and/or salt or hydroxide content. Instrument 151 can optionally also include temperature, pressure, and fluid physical properties instruments, or such instruments can be separately coupled to the respective streams. Instrument 151 can be operatively coupled to the controller 124, which can be further configured to control recycle rate of the water streams 152 to target composition of the eluent stream 108 based, on composition and/or the condition (pressure, temperature, etc.) signals from the instrument 151. A composition instrument 198 (optionally including temperature, pressure, and fluid physical properties) can be coupled to the lithium hydroxide product 199 to monitor for impurities, and can be operatively coupled to the controller 124, which can be further configured to control impurity removal at the brine source 102, the pretreat 105, or the impurity removal process 132 based on signals from the instrument 198.

The need for evaporation in the conversion process 150 can be reduced by using one or more membrane separation units to remove some water prior to the evaporation steps. For example, after converting lithium to lithium hydroxide, the lithium hydroxide stream can be heated to sub-boiling temperatures, for example 200 - 210°F to maximize the solubility limit of lithium hydroxide. The heated lithium hydroxide stream can then be subjected to membrane separation units to remove water as a permeate stream. The non-permeate stream, concentrated in lithium hydroxide, can then be cooled to encourage lithium hydroxide to precipitate, and the precipitated solid can be recovered and dried with reduced energy input.

The use of an impurity removal process in the conversion process 150 provides various lithium-containing water streams that can be used in the eluent stream 108 to optimize composition of the eluent stream 108 for lithium unloading from the absorbent medium. Use of such streams recycles through the impurity removal process further cleaning the downstream process and routing the impurities, most of which come from the brine source 102, to the lithium-depleted brine 106 or back to the brine source 102. These recycle streams also play a crucial role in optimizing fresh water usage. Clean brine streams, produced by downstream processes following impurity removal, are utilized as carrier streams, thereby reducing fresh water consumption in the process.

**Figure 2** is a process diagram summarizing a lithium recovery process 200 according to another embodiment. The lithium recovery process 200 of **Figure 2** is similar to the lithium recovery process 100 of **Figure 1** in many respects, and elements of the process 200 that are the same as elements of the process 100 are labeled using the same numerals. In the process 200, two lithium extractors are used instead of one. Here, a first lithium extractor 204 performs a first lithium extraction process, substantially as described above but to an intermediate lithium concentration to form an intermediate lithium extract stream 210. The intermediate lithium extract stream 210 is routed to the impurity removal unit 132, which yields a clean intermediate lithium extract stream 234.

The stream 234 is routed to a second lithium extractor 206 for concentration to an arbitrary solution concentration of lithium, for example near the solubility limit of lithium chloride, to form the clean lithium extract stream 134. In this case, the lithium-depleted brine 106 is a first lithium-depleted brine, and the second lithium concentrator 206 produces a second lithium-depleted brine 236 that may be recycled to the first lithium extractor 204 for use in the eluent stream 108. As above, the first lithium extractor 204 may also use make-up water from the water source 112 for the eluent stream 108. The second lithium extractor 206 can also use make-up water from the water source 112 or the water recovery 107 as eluent, but also uses recycled water and lithium-containing streams from the downstream concentrator 138 and conversion process 150.

Separating lithium extraction into two stages, with impurity removal between the two stages, allows the second lithium extractor 206 to serve also as a final stage of impurity removal. The intermediate stream 234 may have a low level of impurities that are not removed by the impurity removal unit 132, but the selectivity of the resin in the second lithium extractor 206 will result in very low levels of impurities, if any. Use of two extraction stages 204 and 206 may also be more effective in preventing downstream transfer of impurities, since any impurities that pass through the first extraction stage 204 may be partially or completely removed by the second extraction stage 206. The capacity of the first lithium extractor 204 can also be lower since the total lithium uptake capacity needed is now split between the two extractors 204 and 206. Depending on the brine source used for the process 200, splitting lithium extraction into two stages, with impurity removal between the two stages, may lower the overall capital investment and operating cost needed to accomplish the lithium recovery.

The composition instrument 137 can be used similarly here in conjunction with the controller 124, which can be configured to control the impurity removal unit 132 and distribute the lithium uptake load of the lithium extractors 204 and 206 to target or minimize the level of one or more impurities in the clean stream 134. The controller 124 can control the concentrator 138 to accomplish any impurity removal that might be needed downstream of the second lithium extractor 206. In the process 200, impurities will mostly circulate between the lithium extractors 204 and 206, and the impurity removal unit 132 will remove them to the water recovery process 107 for return to the environment. If the composition instrument 137 detects changing impurity levels, the controller 124 can be configured to perform a hierarchy of control actions, including intensifying impurity removal at the impurity removal unit 132, for example by increasing addition of alkalinity to increase pH at the impurity removal unit 132, increasing flow of eluent stream 108 to reduce solution concentration of impurities entering the impurity removal unit 132, and intensifying permeation of the concentrator 138. Depending on the nature of the impurities detected by the composition instrument 137, the controller 124 may be further configured to control the pretreat unit 105 to increase or decrease intensity of impurity removal upstream of the first lithium extractor 204.

It should be understood that while **Figure 1** and **Figure 2** illustrate example DLE processes, other types of lithium extraction and other techniques for DLE may be used with the brine pre-treatment techniques described herein.

Depending on the nature of the brine, various pre-treatment processes may be useful to remove some of the impurities before the mineral extraction process. Accordingly, brine pre-treatment process that may be used with mineral extraction techniques.

In some aspects, a brine pre-treatment involves a plurality of brine pre-treatment stages. Each brine pre-treatment stage is associated with a brine treatment process that modifies a particular property of the brine. For example, a pre-treatment stage may remove a particular impurity, or type of impurity from the brine, may adjust pH level and/or oxidation-reduction potential (ORP) of the brine, and/or may adjust temperature of the brine. Before performing the brine pre-treatment process for a given brine pre-treatment stage, a measurement of a property (e.g., size or concentration) of the impurity or the brine itself is compared to specified threshold to determine whether to bypass or perform the brine pre-treatment associated with that particular brine pre-treatment stage. In some aspects, the plurality of brine pre-treatment stages are evaluated in a particular order. In some aspects, the order of the brine pre-treatment stages are associated with coarser to finer removal of impurities from the brine.

As used herein, an impurity may refer to a substance removed from a raw brine during a brine pre-treatment process. As used herein, an element of interest may refer to the target substance to be extracted from a process brine (e.g., lithium). In some aspects, an impurity is a substance that may, if not removed from the brine, interfere with the equipment and/or with the extraction of the element of interest.

Accordingly, the brine pre-treatment process is flexible, where the particular brine pre-treatment stages performed on the brine are selected based on the measurements of one or more properties of the brine and on comparison with thresholds selected according to several criteria, such as desired purity level, stringency of the environmental laws, production rates, etc. In this way, the efficiency of the brine pre-treatment and subsequent lithium extraction can be improved.

According to certain aspects, a flexible brine pre-treatment process can be performed before DLE to change one or more properties of the brine, such as removing impurities, adjusting the brine's pH level, adjusting its ORP of the brine, and/or change another property of the brine, which in turn may improve the efficiency and effectiveness of the DLE process.

In some aspects, the flexible pre-treatment process involves multiple pre-treatment stages to adjust one or more properties of the brine.

In some aspects, the flexible brine pre-treatment is based on measurements of one or more properties of the brine. The measurements can be performed to select the required pre-treatment stages from the array of multiple pre-treatment stages in order to optimize the efficiency and effectiveness of the brine pre-treatment and the DLE process.

In the field, brine composition may change due to a combination of natural geological and environmental factors, seasonal shifts, human activities, and contaminations originating from various sources. Further, varying brine samples may contain different levels of specific impurities. Accordingly, the measurements can be used to dynamically select the required brine pre-treatment stages in real-time. In another embodiment, the measurements are used in a design phase of the brine pre-treatment process to design the required pre-treatment stages based on different parameters, including, but not limited to, the location, impurities to be extracted, the equipment used upstream and downstream, operating conditions, requirements and budget, the expected grade and production rate of the final product, and environmental regulations.

In some aspects, one or more properties of the brine is measured before implementing each brine pre-treatment stage. The measured properties are then compared to a specified range(s)/threshold(s). In some aspects, different brine properties and/or different specified ranges/threshold(s) for those properties may be associated with one, or more than one, brine pre-treatment stage. In some aspects, for each different brine pre-treatment stage, a range or combination of brine pre-treatment techniques may be applied.

In some aspects, if the measured value(s) of the one or more properties of the brine are larger the specified range(s)/threshold(s), the respective brine pre-treatment stage(s) is used for the brine. For example, the brine may be routed to the respective brine pre-treatment stage. If the measured value(s) of the one or more properties of the brine is equal to smaller than the specified range(s)/threshold(s), the respective brine pre-treatment stage is not used. For example, the respective brine pre-treatment stage may be bypassed and the brine is routed to a subsequent brine pre-treatment stage.

In some aspects, after performing a brine pre-treatment stage, a second measurement of the one or more properties is performed and compared to the specified range(s)/threshold(s). If the second measurement of the one or more properties of the brine is larger than the specified range, the brine pre-treatment stage may be performed a second time. For example, the brine may be re-routed to the respective brine pre-treatment stage. If the second measurement of the one or more properties of the brine is not within the specified range, a subsequent brine pre-treatment stage may be performed. For example, the brine may be routed to a subsequent brine pre-treatment stage. In some aspects, the measurement and pre-treatment stage is be performed iteratively until the desired value(s) for the one or more properties of the brine is not equal to or smaller than the specified range(s)/threshold(s).

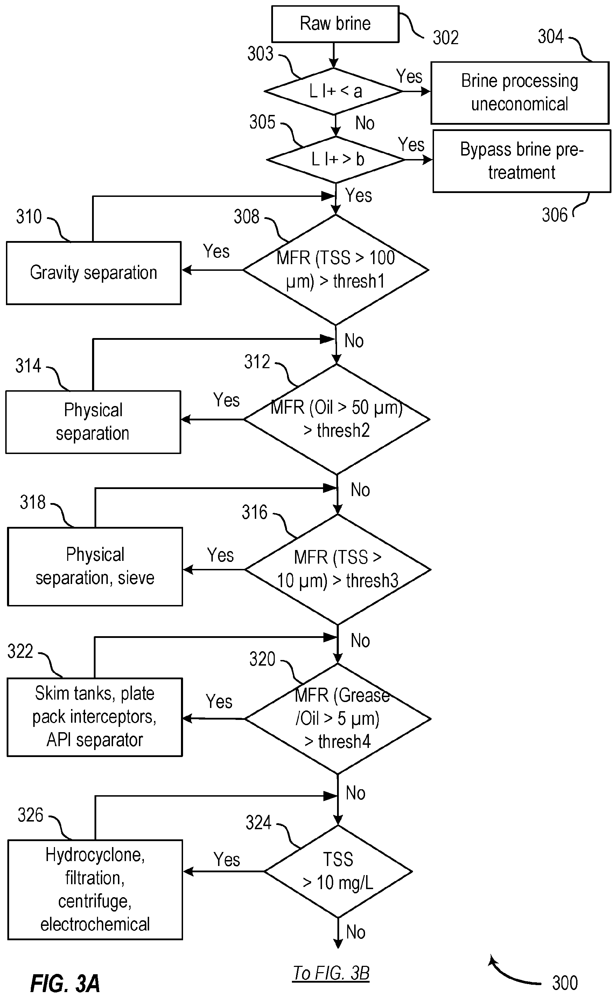

**Figures 3A-3D** illustrate a brine pre-treatment process 300 according to certain aspects. While **Figures 3A-3D** illustrate various examples of the brine pre-treatment stages and their corresponding thresholds. It should be understood that these stages and thresholds are examples, and that different stages with varying orders and different thresholds may be used, for example, based on impurities to be extracted, the equipment used upstream and downstream, operating conditions and requirements, the expected grade and production rate of the final product, and environmental regulations. In addition, while various examples of impurity measurement methods are described herein for the brine pre-treatment process, it should be understood that any suitable method of measurement may be used for determining a particular component or property of the brine.

As shown in **Figure 3A,** at block 302, raw brine may be sampled to determine the physical, chemical, geological, biological, radioactive, and isotopic properties of the raw brine. At block 303, a concentration of lithium ($Li^+$) of the raw brine may be compared to a minimum threshold $a.$ The minimum threshold $a$ may be a lower lithium concentration threshold below which it would be uneconomical to process the raw brine. As shown in **Figure 3A,** at block 304, if the concentration of the lithium ions in the raw brine is below the minimum threshold a, then the brine is not processed. At block 305, the concentration of lithium of the raw brine may be compared to a maximum threshold $b.$ The maximum threshold $b$ may be an upper lithium concentration threshold (e.g., around 1500 mg/L) above which it would be unnecessary to pre-treat the raw brine. As shown in **Figure 3A,** at block 306, if the concentration of the lithium ions in the raw brine is above the maximum threshold $b,$ then the brine pre-pretreatment (and brine extraction) is bypassed. If the concentration of the lithium ions in the raw brine is between the minimum threshold $a$ and the maximum threshold $b,$ then the brine pre-treatment process 300 continues to a first brine pre-treatment stage at block 308.

As shown in **Figure 3A,** at block 308, the brine is tested for a concentration, or mass flow rate (MFR), of total suspended solids (TSS) having a size greater than a predetermined threshold (thresh1), for instance a 100, 150, 500, or 1000 $\mu$m threshold. A mass flow rate for such TSS is measured and compared to a predetermined threshold (thresh2), for instance a 10, 20, 50, or 100 lb/day threshold. Suspended solids may include solid particulate matter in the brine such as sand, silt, clays, proppants, carbonate scales, sulfate scales, corrosion products, sediment, algae, and plankton. Total suspended solids can interfere with the direct extraction of lithium from the brine. For example, suspended solids can foul or block membranes and adsorbent materials reducing their efficiency and lifespan. In addition, can clog filtration systems and other equipment used in the DLE process. Further, discharging brine with high total suspended solid levels into the environment can have negative ecological impacts. Therefore, pre-treatment of the brine to reduce the total suspended solids level may improve the efficiency and reliability of DLE and help to minimize harm to ecosystems and ensuring compliance with environmental regulations.

As shown in **Figure 3A,** where the brine includes a concentration of total suspended solids (larger than thresh1) that exceeds the predetermined threshold (thresh2), a gravity separation (e.g., sedimentation) pre-treatment processing stage is performed on the brine at block 310. Gravity separation is a process that separates lithium-containing minerals or compounds from other materials (e.g., TSS) based on their differences in density-due to the fact that minerals or compounds of different densities will separate naturally when subjected to gravity. Some examples of processing equipment used for gravity separation include, but are not limited to, sand traps, settling tanks, hydrocyclone filtration, and dense media separators. The brine may be introduced into the processing equipment, and gravity is used to separate the lithium-rich compounds or minerals from the gangue (waste) materials. The separation may occur as the material moves through the equipment. The separated lithium-rich concentrate is collected, and the remaining gangue may be typically discarded as waste or further processed to recover any valuable materials it might contain.

After the gravity separation pre-treatment of the brine at block 310, the pre-treatment process 300 may return to the block 308 to check whether the concentration of the total suspended solids level (larger than thresh 1) in the brine pre-treated at stage 310 (that is or derives from the separated lithium-rich concentrate collected from stage 310) is above the predetermined threshold (thresh2). The blocks 308 and 310 may be performed iteratively until the MFR of the total suspended solids (larger than thresh1) in the brine is at or below the predetermined threshold (thresh2). Where the brine includes a concentration of total suspended solids (larger than thresh1) that is at or below the predetermined threshold (thresh2), the gravity separation pre-treatment processing stage is bypassed and the brine pre-treatment process 300 proceeds to block 312.

As shown in **Figure 3A,** at block 312, the brine is tested for a concentration, or MFR, of oil droplets having a size greater than a threshold (thresh3, for instance a 50,

100, or 150 $\mu$m threshold). A concentration or MFR for such oil droplets is measured and compared to a predetermined threshold (thresh4), for instance a 50, 100, 500, 1000, or 2000 mg/L threshold. The presence of oil droplets in brine may interfere with subsequent separation techniques. For example, oil droplets in the brine may interference with solvent extraction, ion exchange, and membrane processes by coalescing with organic phases or adsorbents, which may reduce the efficiency of the processes, may cause blockages, and may foul the equipment used for those separation processes. Oil droplets can coat surfaces and interfere with mass transfer processes, slowing down the extraction rate of lithium ions from the brine, thereby resulting in lower lithium recovery and increased processing time. In addition, discharging oil-contaminated brine into the environment can have negative environmental impacts, potentially causing pollution and harming ecosystems. Therefore, pre-treatment of the brine to remove oil droplets may improve the efficiency and reliability of lithium extraction and help to minimize harm to ecosystems and ensuring compliance with environmental regulations.

In some aspects, the oil in the brine is measured using a gravimetric technique.

In some aspects, the oil in the brine is measured using a titration technique that involves mixing a volume of the brine is mixed with a solvent to extract the oil. Then, an indicator solution is added, and the mixture is titrated with a standard solution of a chemical reagent (e.g., such as potassium hydroxide or sulfuric acid) until a color change indicates the endpoint. The volume of the titrant used is proportional to the oil concentration.

In some aspects, the oil in the brine is measured using a spectroscopy technique. An infrared (IR) spectroscopy technique involves analyzing the composition of a sample by measuring the absorption of infrared light at specific wavelengths. Oil has characteristic absorption bands in the IR spectrum, allowing for quantitative analysis. An ultraviolet-visible (UV-Vis) spectroscopy technique involves measuring the specific absorption characteristics in the UV or visible range. A fluorescence spectroscopy technique involves measuring fluorescence of oil compounds when exposed to certain wavelengths of light to quantify the concentration of the oil compounds in the brine.

In some aspects, the oil in the brine is measured using a chromatography technique. A gas chromatography or high-performance liquid chromatography (HPLC) technique involves injecting a sample of the brine into a chromatograph, where the components are separated based on their chemical properties and detected using a suitable detector, which can provide detailed information about the composition of the oil in brine.

In some aspects, the oil in the brine is measured using a mass spectrometry technique to identify and quantify specific oil components in brine with high sensitivity and selectivity. Mass spectrometry works by ionizing the molecules and measuring their mass-to-charge ratio.

In some aspects, the oil in the brine is measured using a solvent extraction technique.

As shown in **Figure 3A,** where the brine includes a concentration of oil droplets (larger than thresh3) that exceeds the predetermined threshold (thresh4), a physical separation pre-treatment processing stage is performed on the brine at block 314. Physical separation processes may include, but are not limited to, gravity separation, centrifugation, filtration, electrostatic separation, electrocoagulation, flotation (e.g., dissolved gas flotation and induced gas floatation), and coalescence, or a combination thereof.

Centrifugation uses centrifuges that spin at high speeds to separate materials of different densities. For example, a hydrocyclone is a conical device that use centrifugal force to separate oil and water. The oil and water are introduced tangentially, and the centrifugal force causes them to separate with the oil moving towards the center and the water towards the outer edge.

Filtration separation (e.g., media filtration) involves passing the brine through a porous medium (e.g., a filter) that retains impurities, allowing the clean brine to pass through. Membrane filtration processes use semipermeable membranes with specific pore sizes to physically filter out targeted materials (e.g., oil droplets) from the brine. Different types of membrane processes, such as microfiltration, ultrafiltration, and nanofiltration, can be employed.

Electrostatic separation utilizes differences in the electrical conductivity and charge properties of particles to separate them.

Electrocoagulation is an electrochemical process uses electrically generated coagulants to destabilize and aggregate oil droplets, making them easier to remove through sedimentation or flotation.

Flotation involves the attachment of air bubbles to particles, causing them to float to the surface, where they can be collected and removed. Dissolved air flotation (DAF) is a process that introduces air bubbles into the brine, which attach to the oil droplets, causing them to rise to the surface and form a froth layer. This froth can then be skimmed off to remove the oil.

Coalescers are devices that facilitate the merging or coalescence of small oil droplets into larger ones. These larger droplets are easier to separate from the brine. For example, plate separators, also known as corrugated plate interceptors, parallel plate interceptors or plate pack interceptors, use a series of inclined plates to trap and separate oil droplets from the brine. As the brine flows over the plates, oil droplets coalesce and rise to the surface.

After the physical separation pre-treatment of the brine at block 314, the pre-treatment process 300 may return to the block 312 to check whether the concentration of the oil droplets (larger than thresh3) in the brine pre-treated at stage 312 are above the predefined threshold (thresh4). The blocks 312 and 314 may be performed iteratively until the concentration of oil droplets (larger than thresh3) in the brine is at or below the predetermined threshold (thresh4). Where the brine includes a concentration of oil droplets (larger than thresh3) that is at or below the predetermined threshold (thresh4), the physical separation pre-treatment processing stage is bypassed and the brine pre-treatment process 300 proceeds to block 316.

As shown in **Figure 3A,** at block 316, the brine is tested for a concentration, or MFR, of TSS having a size greater than a threshold (thresh5), for instance a 5, 10 or 50 $\mu$m threshold. A mass flow rate for such TSS is measured and compared to a predetermined threshold (thresh6), for instance a 1, 5 or 10 lb/day threshold. Pretreatment of the brine to reduce the concentration of the TSS larger than thresh5 at block 318, after the pretreatment of the brine to reduce the concentration the TSS larger than the coarser thresh1 at block 310, allows for further finer granularity processing of the TSS from the brine after the removal of the coarser TSS.

As shown in **Figure 3A,** where the brine includes a concentration of TSS (larger than thresh5) that exceeds the predetermined threshold (thresh6), a physical separation pre-treatment processing stage is performed on the brine at block 318. In some aspects, a sieve is used for the physical separation of the finer TSS. Sieving is a technique that can effectively separate TSS from the brine based on particle size. The mesh size of a sieve refers to the number of openings or holes per linear inch or per square inch. The choice of sieve depends on the desired level of particle size separation. Finer mesh sizes are used to separate smaller particles, while coarser mesh sizes are used for larger particles. When the brine containing the TSS passes through the sieve, the brine passes through the openings in the sieve, while the TSS are trapped on the surface of the sieve.

After the physical separation pre-treatment of the brine at block 318, the pre-treatment process 300 may return to the block 316 to check whether the concentration of the TSS (larger than thresh5) in the brine pre-treated at stage 316 is still above the predetermined threshold (thresh6). The blocks 316 and 318 may be performed iteratively until the concentration of the TSS (larger than thresh5) in the brine is at or below the predetermined threshold (thresh6). Where the brine includes a concentration of TSS (larger than thresh5) that is at or below the predetermined threshold (thresh6), the physical separation pre-treatment processing stage is bypassed and the brine pre-treatment process 300 proceeds to block 320.

As shown in **Figure 3A,** at block 320, the brine is tested for a concentration or MFR of grease droplets and finer oil droplets having a size that exceeds a threshold (thresh7), for instance greater than a 5, or 10 $\mu$m threshold. A mass flow rate for such grease and oil droplets is measured and compared to a predetermined threshold (thresh8), for instance 5, 10, 20, 40, 60, or 100 mg/L threshold.

As shown in **Figure 3A,** where the brine includes a concentration of grease or oil droplets (larger than thresh?) that exceeds the predetermined threshold (thresh8), a separation pre-treatment processing stage is performed on the brine at block 322. The separation of the grease and/or oil droplets from the brine may use skim tanks, plate pack interceptors, and/or American Petroleum Institute (API) separators.

A skim tank, also known as an oil skimming tank or oil skimmer, is a piece of equipment used for separating oil and grease droplets from a liquid, such as brine. A skim tank employs a physical separation process to remove the hydrophobic (water-repellent) oil and grease from the hydrophilic (water-attracting) brine. A skim tank may be a rectangular or cylindrical tank equipped with various components designed for the separation process, such as overflow weirs, effluent outlets, and baffles.

Skim tanks may include, but are not limited to, floating oil skimmer, belt skimmers, and drum skimmers. A floating oil skimmer includes a floating device that moves along the surface of the liquid, collecting the floating oil and grease. The floating oil skimmer is equipped with skimming belts, discs, or tubes that physically remove the oil and grease from the surface. Belt skimmers use a loop of material (e.g., a belt or tube) that passes through the surface of the liquid, collecting the oil and grease. Drum skimmers are similar to belt skimmers but use a rotating drum to collect oil from the surface. As the drum rotates, oil adheres to the surface and is scraped off for removal.

The skimming mechanism collects the oil and grease from the surface of the brine. The collected oil and grease may be deposited into a separate container or channel for further processing, recycling, or disposal. The clarified brine, now separated from most of the oil and grease, exits the skim tank through an effluent outlet (e.g., located at the bottom of the tank). Baffles may be installed within the skim tank to create a controlled flow pattern. Baffles help prevent turbulence and ensure that the separated oil and grease remain on the surface for efficient skimming.

A plate pack interceptor may include a tank or a chamber equipped with a series of closely spaced inclined plates, which may be made of corrugated or coalescing materials. These plates create a series of narrow channels or passages through which the contaminated brine flows. The contaminated brine, which contains oil and grease droplets, may be introduced into the plate pack interceptor through an inlet pipe. As the brine flows through the narrow channels between the plates, the oil and grease droplets interact with the plates' surfaces. The inclined plates in the interceptor provide a large surface area for the oil and grease droplets to come into contact with. The corrugated or coalescing design of the plates promotes coalescence, in which smaller droplets combine to form larger droplets. As the oil and grease droplets coalesce and become larger, they become less buoyant and begin to rise due to differences in density and adhere to the surface of the plates. This rising and adherence process separates the oil and grease from the brine. The now cleaner brine, with reduced oil and grease content, may continue to flow through the narrow channels and exit the plate pack interceptor through an outlet pipe. The separated oil and grease, adhered to the plates, accumulate and form a layer on the surface of the plates. This accumulated oil and grease can be removed, either manually or through automated systems, for proper disposal or recycling.

An API separator is a specialized piece of equipment used in the oil and gas industry to separate oil and grease droplets from produced water, wastewater, or other aqueous mixtures. An API separator is a gravity separation device that based on the specific gravity difference between grease and oil droplets and brine.

After the separation pre-treatment of the brine at block 322, the pre-treatment process 300 may return to the block 320 to check whether the concentration of grease and oil droplets (larger than thresh?) in the brine pre-treated at block 322 is still above the predetermined threshold (thresh8). The blocks 320 and 322 may be performed iteratively until the concentration of grease and oil droplets (larger than thresh?) in the brine is at or below the predetermined threshold (thresh8). Where the brine includes a concentration of grease or oil droplets (larger than thresh?) that is at or below the predetermined threshold (thresh8), the separation pre-treatment processing stage is bypassed and the brine pre-treatment process 300 proceeds to block 324.

As shown in **Figure 3A,** at block 324, the brine is tested for TSS having a size greater than a predetermined threshold, for instance a 0.5, 1, 3 or 5 $\mu$m threshold (thresh9). A mass flow rate for such TSS is measured and compared to a predetermined threshold (thresh10), for instance 0.5, 1, or 5 lb/day threshold.

As shown in **Figure 3A,** where the brine includes a concentration of TSS (larger than thresh9) that exceeds the predetermined threshold (thresh10), another physical separation pre-treatment processing stage is performed on the brine at block 326 that includes methods such as hydrocyclone separation, filtration, centrifuge separation, and/or electrochemical separation, or a combination thereof.

After the hydrocyclone separation, filtration, centrifuge separation, and/or electrochemical separation pre-treatment of the brine at block 326, the pre-treatment process 300 may return to the block 324 to check whether the concentration of TSS (larger than thresh9) in the brine pre-treated at stage 326 are above the predetermined threshold (thresh10). The blocks 324 and 326 may be performed iteratively until the TSS concentration (larger than thresh9) in the brine is at or below the predetermined threshold (thresh10). Where the brine includes TSS concentration (larger than thresh9) that is at or below the predetermined threshold (thresh10), the hydrocyclone separation, filtration, centrifuge separation, and/or electrochemical separation pre-treatment processing stage is bypassed and the brine pre-treatment process 300 proceeds to block 328.

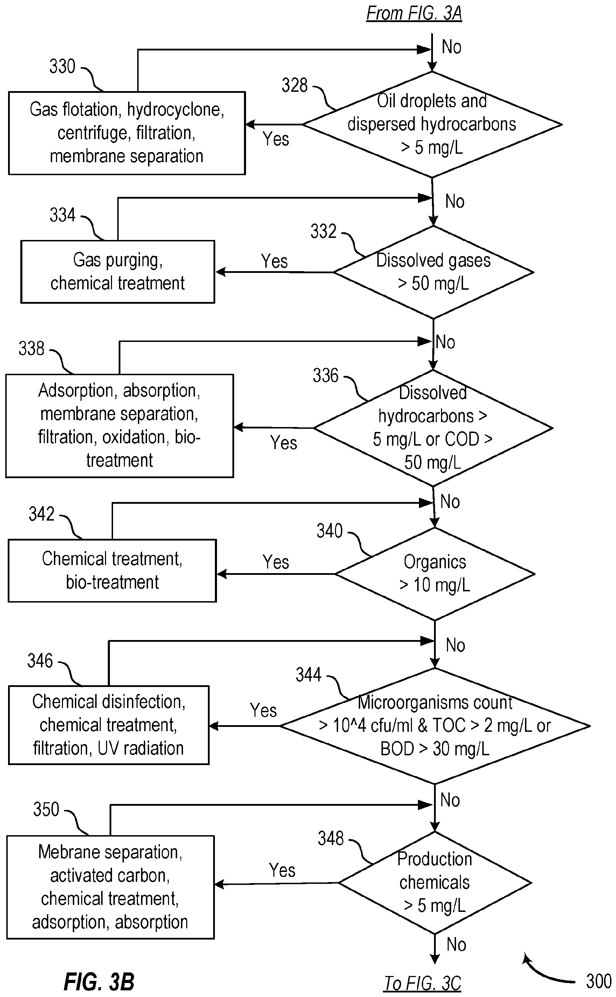

As shown in **Figure 3B,** at block 328, the brine is tested for a concentration or MFR of dispersed hydrocarbons and ultra small oil droplets having a concentration greater than a threshold (thresh11), for instance a 1, 5, or 10 mg/L threshold. Dispersed hydrocarbons are hydrocarbon compounds that exist as ultra small droplets or suspended particles within the water. These hydrocarbons are not fully mixed or dissolved in the water; instead, they are dispersed throughout it. Examples of dispersed hydrocarbons include heavier alkylated phenols, such as $C_6$-$C_9$ alkylated phenols.

As shown in **Figure 3B,** where the brine includes dispersed hydrocarbons and ultra small oil droplets concentration that exceeds the predetermined threshold (thresh11), a pre-treatment stage is performed on the brine at block 330 that includes methods such as gas flotation separation (e.g., dissolved gas flotation and induced gas floatation), hydrocyclone separation, centrifuge separation, filtration, membrane separation, or a combination thereof.

After the gas flotation separation, hydrocyclone separation, centrifuge separation, filtration, and/or membrane separation pre-treatment of the brine at block 330, the pre-treatment process 300 may return to the block 328 to check whether dispersed hydrocarbons and ultra small oil droplets concentration in the brine pre-treated at block 330 is above the predetermined threshold (thresh 11). The blocks 328 and 330 may be performed iteratively until the dispersed hydrocarbons and ultra small oil droplets concentration in the brine is at or below the predetermined threshold (thresh11). Where the brine includes dispersed hydrocarbons and ultra small oil droplets concentration that is at or below the predetermined threshold (thresh11), the gas flotation separation, hydrocyclone separation, centrifuge separation, filtration, and/or membrane separation pre-treatment processing stage is bypassed and the brine pre-treatment process 300 proceeds to block 332.

In some aspects, the concentration of the dispersed hydrocarbons in the brine is measured using a gravimetric technique.

In some aspects, the concentration of the dispersed hydrocarbons in the brine is measured using a chromatography technique such gas chromatography and HPLC.

In some aspects, the concentration of the dispersed hydrocarbons in the brine is measured using a spectroscopy technique such IR and UV-vis. In some aspects, the concentration of the dispersed hydrocarbons in the brine is measured using a nuclear magnetic resonance (NMR) spectroscopy technique.

As shown in **Figure 3B,** at block 332, the brine is tested for dissolved gases having a concentration greater than a threshold (thresh12), for instance a 10, 50, or 100 mg/L threshold. The dissolved gases in the brine may include toxic, flammable, and corrosive gases (e.g., $H_2S$,

$CO_2$, $O_2$, $CH_4$, and $H_2$). The presence of such dissolved gases in the brine may present safety hazards, environment concerns, operation challenges, and regulatory issues. Toxic gases dissolved in brine can pose health risks to workers and the surrounding environment. Exposure to high concentrations of toxic gases can lead to respiratory problems, chemical burns, and other health issues. Flammable gases in brine can create explosion and fire hazards. Discharging brine with toxic dissolved gases into the environment can lead to contamination of water bodies, soil, and ecosystems. Corrosive gases can damage equipment and pipelines. If leaks occur, these gases may escape into the environment, causing localized environmental damage. Corrosive gases can corrode equipment and infrastructure used in the lithium extraction process, leading to increased maintenance costs, reduced equipment lifespan, and potential safety hazards. Therefore, pre-treatment of the brine to reduce the dissolved gases may improve the efficiency and reliability of lithium extraction, minimize harm to workers and ecosystems, and help to meet environmental regulations.

In some aspects, the concentration of the dissolved gases in the brine is measured using gas chromatography.

In some aspects, the concentration of the dissolved gases in the brine is measured using titration.

In some aspects, the concentration of the dissolved gases in the brine is measured using gas instruments, such as colorimetric detection tubes, gas-sensitive electrodes or solid-state instruments, can be used to detect specific gases in brine. These instruments work by changing their electrical properties in response to the presence of a particular gas, allowing for the measurement of gas concentration. In some aspects, the concentration of the dissolved gases in the brine is measured using electrochemical instruments with electrodes to detect changes in electrical current or potential caused by the reaction of the gas with specific chemicals or materials. The magnitude of the change is proportional to the concentration of the gas.

In some aspects, the concentration of the dissolved gases in the brine is measured using gas dissolution and pressure measurement techniques. The solubility of gases in liquids is temperature and pressure-dependent. By measuring the pressure change after introducing the brine to a closed system, the concentration of dissolved gases can be indirectly determined.

In some aspects, the concentration of the dissolved gases in the brine is measured using infrared spectroscopy.

In some aspects, the concentration of the dissolved gases in the brine is measured using mass spectrometry, and/or pH measurements.

As shown in **Figure 3B,** where the brine includes dissolved gases concentration that exceeds the predetermined threshold (thresh12), a gas purging chemical pre-treatment processing stage is performed on the brine at block 334. A gas purging chemical treatment may separates the unwanted dissolved gases from the brine by introducing a different chemical, such as an inert or non-reactive gas like nitrogen or air, into the brine. With sparging gas purging technique, gas is introduced into the brine through small bubbles or a diffuser at the bottom of a vessel or tank. As the gas rises through the brine, it carries the dissolved gases with it to the surface, where they are released. In a chemical gas purging treatment, specific chemical reagents or additives (e.g., scavenging agents, carbonate precipitants, amine-based based acid gas removal solutions) are introduced into the brine to react with and remove the dissolved gases from the brine. The choice of chemical treatment depends on the type of gas to be removed and other parameter related to the rest of the process, in particular making sure it is not detrimental to the DLE process.

After the gas purging/chemical pre-treatment of the brine at block 334, the pre-treatment process 300 may return to the block 332 to check whether the dissolved gases concentration in the brine pre-treated at block 334 is above the predetermined threshold (thresh12). The blocks 332 and 334 may be performed iteratively until the dissolved gases concentration in the brine is at or below predetermined threshold (thresh12). Where the brine includes dissolved gases concentration that is at or below the predetermined threshold (thresh12), the gas purging chemical pre-treatment processing stage is bypassed and the brine pre-treatment process 300 proceeds to block 336.

As shown in **Figure 3B,** at block 336, the brine is tested for dissolved hydrocarbons having a concentration greater than a threshold (thersh13), for instance 1, 5, or 10 mg/L threshold and/or a chemical oxygen demand (COD) greater than a threshold (thresh14), for instance 50, 100, 150, or 200 mg/L threshold. The dissolved hydrocarbons are hydrocarbon compounds that are partially to fully mixed at the molecular level with water. A few examples of dissolved hydrocarbons are volatile aromatic compounds, BTEX (benzene, toluene, ethyl benzene, and xylene), phenols, PHA (polycyclic aromatic hydrocarbons), aliphatic hydrocarbons, and NPD (naphthalene, phenanthrene, and dibenzothiophene). The dissolved hydrocarbons in the brine can chemically interact with lithium extraction reagents or interfere with the physical separation methods used in the DLE process, including subsequent pre-treatment stages, which lead to reduced lithium recovery efficiency and overall yield. Further, the dispersed hydrocarbons in the brine can lead to the formation of emulsions. These emulsions are challenging to break and can make it difficult to separate lithium from the brine using methods such as membrane filtration, solvent extraction, or adsorption. Further, the dispersed hydrocarbons in the brine can foul and clog equipment used in the lithium extraction process, such as filters, membranes, and extraction columns, resulting in increased maintenance, downtime, and operational costs. In addition, discharging brine with dispersed hydrocarbons into the environment can have environmental impacts. Hydrocarbons can contaminate surface waters, soils, and aquatic ecosystems, posing a risk to the environment and local wildlife. Moreover, many regions have strict environmental regulations governing the discharge of hydrocarbon-contaminated water and failure to comply with these regulations can result in legal and financial penalties. Therefore, pre-treatment of the brine to reduce the dispersed hydrocarbons in the brine may improve the efficiency and reliability of DLE and help to minimize harm to ecosystems and ensuring compliance with environmental regulations.

In some aspects, the concentration of the dispersed hydrocarbons in the brine is measured using a gravimetric technique.

In some aspects, the concentration of the dispersed hydrocarbons in the brine is measured using titration.

In some aspects, the concentration of the dispersed hydrocarbons in the brine is measured using a solvent technique.

In some aspects, the concentration of the dispersed hydrocarbons in the brine is measured using a spectroscopy technique. In some aspects, the concentration of the dispersed hydrocarbons in the brine is measured using a nuclear magnetic resonance (NMR) spectroscopy technique.

As shown in **Figure 3B,** where the brine includes dissolved hydrocarbon and/or COD concentration that exceeds the predetermined threshold(s) (thresh13 and/or thersh14), an adsorption, absorption, membrane separation, filtration, advanced oxidation process, and/or biological treatment pre-treatment processing stage is performed on the brine at block 338.

After the adsorption, absorption, membrane separation, filtration, advanced oxidation process, and/or biological treatment pre-treatment of the brine at block 338, the pre-treatment process 300 may return to the block 336 to check whether the dispersed hydrocarbon and/or COD concentration in the brine pre-treated at block 338 is above the predetermined threshold(s) (thresh13 and/or thresh14). The blocks 336 and 338 may be performed iteratively until the dispersed hydrocarbon concentration in the brine is at or below the predetermined threshold(s) (thresh13 and/or thresh14). Where the brine includes dispersed hydrocarbon concentration that is at or below the predetermined threshold(s) (thresh13 and/or thresh14), the adsorption and/or membrane separation pre-treatment processing stage is bypassed and the brine pre-treatment process 300 proceeds to block 340.

As shown in **Figure 3B,** at block 340, the brine is tested for organics, including acids or other water-soluble organics, having a concentration greater than a threshold (thresh15), for instance a 1, 5, 10, or 100 mg/L threshold. The organic acids in the brine may include, but are not limited to, formic acid, acetic acid, hexanoic acid, butanoic acid, propanoic acid, and pentanoic acid. The presence of organic acids in the brine may pose similar problems as the presence of acidic dispersed gases in the brine. Therefore, pre-treatment of the brine to reduce the organic acid concentration in the brine may improve the efficiency and reliability of DLE and help to minimize harm to ecosystems and ensuring compliance with environmental regulations.

In some aspects, the concentration of the organics in the brine is measured using a spectroscopy technique.

In some aspects, the concentration of the organics in the brine is measured using a titration technique.

In some aspects, the concentration of the organics in the brine is measured using a chromatography technique. In some aspects, an ion chromatography technique, a form of liquid chromatography, is used.

In some aspects, the concentration of the organics in the brine is measured using a colorimetric or spectrophotometric technique that involves chemical reactions that produce a colored product, the intensity of which is proportional to the concentration of the organic acid in the brine.

In some aspects, the concentration of the organics in the brine is measured using a mass spectrometry technique.

In some aspects, the concentration of the organics in the brine is measured using an enzymatic assay technique, where enzymes that specifically react with the target acid are introduced. The change in enzyme activity can be correlated with the concentration of the organic acid in the brine.

As shown in **Figure 3B,** where the brine includes organics concentration that exceeds the predetermined threshold (thresh15), a chemical and/or biological pre-treatment processing stage is performed on the brine at block 342. The organic acids in the brine may be neutralized by introducing alkaline reagents (e.g., lime or sodium hydroxide) to the brine. The organic acids may be separated from the brine by introducing coagulants (e.g., aluminum sulfate or ferric chloride), adsorbents (e.g., activated carbon or ion exchange resins), and/or precipitants (e.g., metal salts) to the brine, making the organic acids easier to remove by sedimentation or filtration. The organic acids may be separated from the brine by introducing solvents to the brine to selectively extract the organic acids from the brine, leaving behind other components. The organic acids may be separated from the brine by introducing oxidizing agents (e.g., hydrogen peroxide or ozone) to the brine to break down the organic acids into simpler compounds that easier to remove.

After the chemical and/or biological pre-treatment of the brine at block 342, the pre-treatment process 300 may return to the block 340 to check whether the organics concentration in the brine pre-treated at block 342 is above the predetermined threshold (thesh15). The blocks 340 and 342 may be performed iteratively until the organic acids concentration in the brine is at or below the predetermined threshold (thresh15). Where the brine includes organic acids concentration that is at or below the predetermined threshold (thresh15), the chemical pre-treatment processing stage is bypassed and the brine pre-treatment process 300 proceeds to block 344.

As shown in **Figure 3B,** at block 344, the brine is tested for microorganisms such as extracellular polymeric substance forming bacteria, sulfate reducing bacteria, nitrate reducing bacteria, metal oxidizing bacteria, acid producing bacteria, and sulfur oxidizing bacteria, having a concentration greater than a threshold (thresh16), for instance a $10^4$, $5 \times 10^4$, or $10^5$ colony forming unit (cfu)/ml threshold, and/or a total organic carbon (TOC) having a concentration greater than a threshold (thresh17), for instance a 1, 2, or 5 mg/L threshold, and/or a biochemical oxygen demand (BOD) having a concentration greater than a threshold (thresh18), for instance a 20, 30, or 50 mg/L threshold. Microorganisms can accumulate on surfaces, including pipes, pumps, and filtration membranes, reducing flow rates, blocking passages, corrosion of metallic parts and equipment, and decreasing the overall efficiency of the extraction process, and resulting in increased maintenance and operational costs. Further, microorganisms may adsorb lithium ions or create a physical barrier, preventing efficient contact between the lithium-containing brine and the extraction agents or membranes used in the lithium extraction process, leading to reduced lithium recovery. In addition, microorganisms can influence the microbial ecology of the brine. Their metabolic activities may alter the chemical composition of the brine, affecting factors such as pH, salinity, and the concentrations of other ions, which can further complicate the lithium extraction process. Therefore, pre-treatment of the brine to reduce the microorganisms may improve the efficiency and reliability of lithium extraction and help to minimize harm to ecosystems and ensuring compliance with environmental regulations.

In some aspects, the concentration of bacteria in the brine is measured using a commercial test kit.

In some aspects, the concentration of bacteria in the brine is measuring using a plate counting technique that involves spreading a sample of the brine onto an appropriate agar medium. The bacteria grow as cells or colonies, and are counted to estimate bacterial concentration.

In some aspects, the concentration of bacteria in the brine is measured using a fluorescent staining technique that involves using fluorescent dyes to label bacteria. Epifluorescence microscopy or flow cytometry can then be used to count and measure the size of fluorescent particles, allowing estimation of the bacterial concentration.

In some aspects, the concentration of bacteria in the brine is measured using a colorimetry, spectrophotometry, or HPLC technique.

In some aspects, the concentration of bacteria in the brine is measured using a viscosity technique.

Bacteria can increase the viscosity of a solution. Viscosity measurements of the brine can be correlated to bacterial concentration in the brine.

In some aspects, the concentration of bacteria in the brine is measured using a turbidity technique that involves measuring the relative clarity of liquid when a light is shined through a sample of the liquid.

As shown in **Figure 3B,** where the brine includes a concentration of bacteria, TOC, and/or BOD that exceeds the predetermined threshold(s) (thresh16, thresh17, and/or thresh18), a chemical disinfection, chemical treatment, filtration, and/or ultraviolet (UV) light radiation pre-treatment processing stage is performed on the brine at block 336. Chemical disinfection removes the bacteria from the brine by introducing chemicals to the brine that kill or inhibit the growth of the bacteria. A chemical treatment to change the pH of the brine can make the brine unfavorable for the growth of some bacteria.

UV disinfection may use a UV lamp(s) enclosed in a protective chamber made of materials that allow UV light (e.g., UV-C light) to pass through. The brine containing bacteria is passed through the UV disinfection system. As the brine flows through the chamber, it is exposed to intense UV-C light. UV-C light has a specific wavelength (e.g., around 254 nm) that is absorbed by the DNA of the bacteria. The absorbed UV energy damages the DNA structure, causing the formation of thymine dimers, which are molecular bonds between adjacent thymine bases in the DNA. These dimers disrupt the normal functioning of the DNA and prevent the bacteria from reproducing. As a result, the bacteria become inactive.

After the chemical disinfection, filtration, and/or UV radiation pre-treatment of the brine at block 346, the pre-treatment process 300 may return to the block 344 to check whether the concentration of bacteria, the TOC, and/or the BOD in the brine pre-treated at block 346 exceeds the predetermined threshold(s) (thresh16, thresh17, and/or thresh18). The blocks 344 and 346 may be performed iteratively until the predetermined threshold(s) is satisfied. Where the brine satisfies the predetermined threshold(s), the chemical disinfection, chemical treatment, filtration, and/or UV radiation pre-treatment processing stage is bypassed and the brine pre-treatment process 300 proceeds to block 348.

As shown in **Figure 3B,** at block 348, the brine is tested for production chemicals having a concentration greater than a threshold (thresh19), for instance a 10, 15, or 20 mg/L threshold. The production chemicals in the brine may include, but are not limited to, corrosion inhibitors, scale inhibitors, hydration inhibitors, demulsifiers, biocides, biocide enhancers, flocculants, antifoams, and surfactants. Pre-treatment of the brine to reduce the production chemicals may improve the efficiency and reliability of lithium extraction and help to minimize harm to ecosystems and ensuring compliance with environmental regulations.

In some aspects, the concentration of production chemicals in the brine is measured using a titration technique.

In some aspects, the concentration of production chemicals in the brine is measured using a spectrophotometry technique.

In some aspects, the concentration of production chemicals in the brine is measured using a chromatography technique.

In some aspects, the concentration of production chemicals in the brine is measured using electrodes (e.g., ion-selective electrodes) and/or instruments.

In some aspects, the concentration of production chemicals in the brine is measured using a conductivity technique that involves measuring the electrical conductive of the brine, which can be correlated to the concentration of production chemical ions in the solution.

In some aspects, the concentration of production chemicals in the brine is measured using a mass spectrometry technique.

As shown in **Figure 3B,** where the brine includes production chemical concentration that exceeds the predetermined threshold (thresh19), a membrane separation, activated carbon, chemical treatment, adsorption, and/or absorption processing stage is performed on the brine at block 350.

After the membrane separation, activated carbon, chemical treatment, adsorption, and/or absorption of the brine at block 350, the pre-treatment process 300 may return to the block 348 to check whether the production chemical concentration in the brine pre-treated at block 350 is above the predetermined threshold (thresh19). The blocks 348 and 350 may be performed iteratively until the production chemical concentration in the brine is at or below the predetermined threshold (thresh19). Where the brine includes production chemical concentration that is at or below the predetermined threshold (thresh19), the membrane separation, activated carbon, and/or chemical pre-treatment processing stage is bypassed and the brine pre-treatment process 300 proceeds to block 352.

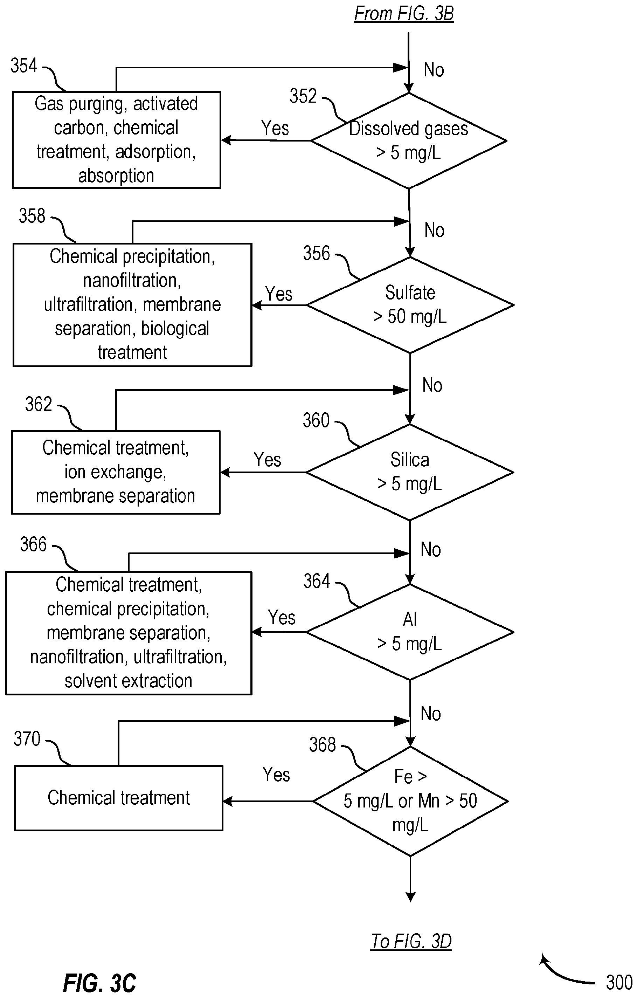

As shown in **Figure 3C,** at block 352, the brine is tested for dissolved gases having a concentration greater than a predetermined threshold (thresh20), which may be 5 mg/L threshold.

As shown in **Figure 3C,** where the brine includes dissolved gases concentration that exceeds the predetermined threshold (threshold20), a gas purging, activated carbon, and/or chemical pre-treatment processing stage is performed on the brine at block 354.

After the gas purging, activated carbon, chemical treatment, adsorption, and/or absorption of the brine at block 354, the pre-treatment process 300 may return to the block 352 to check whether the dissolved gases concentration in the brine pre-treated at block 354 is above the predetermined threshold (thresh20). The blocks 352 and 354 may be performed iteratively until the dissolved gases concentration in the brine is at or below the predetermined threshold (thresh20). Where the brine includes dissolved gases concentration that is at or below the predetermined threshold (thresh20), the gas purging, activated carbon, chemical treatment, adsorption, and/or absorption processing stage is bypassed and the brine pre-treatment process 300 proceeds to block 356.

As shown in **Figure 3C,** at block 356, the brine is tested for sulfate having a concentration greater than a predetermined threshold (thresh21), for instance a 50, 100, or 150 mg/L threshold. Sulfate is a common precipitant in many treatment processes. Pre-treatment of the brine to reduce the concentration of sulfate may help avoid interference with the later remove of other metals from the brine.

As shown in **Figure 3C,** where the brine includes concentration of sulfate that exceeds the predetermined threshold (threshold21), chemical precipitation, nanofiltration, ultrafiltration, membrane separation, and/or biological treatment processing stage is performed on the brine at block 358.

After the chemical precipitation, nanofiltration, ultrafiltration, membrane separation, and/or biological treatment of the brine at block 358, the pre-treatment process 300 may return to the block 356 to check whether the sulfate concentration in the brine pre-treated at block 358 is above the predetermined threshold (thresh21). The blocks 356 and 358 may be performed iteratively until the sulfate concentration in the brine is at or below the predetermined threshold (thresh21). Where the brine includes sulfate concentration that is at or below the predetermined threshold (thresh21), the chemical precipitation, nanofiltration, ultrafiltration, membrane separation, and/or biological treatment processing stage is bypassed and the brine pre-treatment process 300 proceeds to block 360.

As shown in **Figure 3C,** at block 360, the brine is tested for silica having a concentration greater than a predetermined threshold (thresh22), for instance a 1, 5, or 10 mg/L threshold. This can reduce the purity of the extracted lithium. Further, silica can precipitate out of solution under certain conditions, particularly when the brine is subjected to changes in temperature, pressure, or pH. This can lead to the formation of scale deposits in processing equipment, pipelines, and wells, reducing the efficiency of equipment and necessitate costly maintenance and cleaning. Further, silica can foul or block the membranes, reducing their efficiency and lifespan, leading to increased operating costs and downtime. In addition, the disposal of brine containing high levels of silica can have environmental implications. Silica can accumulate in discharge waters, potentially causing environmental harm or violating regulatory limits. Therefore, pre-treatment of the brine to reduce the silica concentration in the brine may improve the efficiency and reliability of lithium extraction and help to minimize harm to ecosystems and ensuring compliance with environmental regulations.

As shown in **Figure 3C,** where the brine includes silica concentration that exceeds the predetermined threshold (thresh22), a chemical treatment, ion exchange (e.g., selective extraction of silica in the brine by replacing silica ions in the brine by other ions present in a ion exchange media such as an insoluble solid), and/or membrane separation (e.g., selective extraction of silica ions using a membrane that allows passage of silica ions but blocks other ions and/or blocks the passage of silica ions but allows passage of other ions) processing stage is performed on the brine at block 362.

In some aspects, the chemical treatment at block 362 is a coagulation chemical treatment.

In some aspects, the chemical treatment at block 362 is a flocculation chemical treatment. Flocculation may involve adding flocculants to the brine. The flocculants are chemicals that promote the aggregation of fine particles and impurities into larger, clumped masses called flocs. Flocculants may include, but are not limited to, polymers or organic compounds.

In some aspects, the chemical treatment at block 362 is an activated alumina chemical pre-treatment. Activated alumina is a highly porous form of aluminum oxide $(Al_2O_3)$ that may be used to selectively capture impurities from the brine.

In some aspects, the chemical treatment at block 362 is a combination of two or more of the techniques mentioned hereinabove.

After the chemical treatment, ion exchange, and/or membrane separation of the brine at block 362, the pre-treatment process 300 may return to the block 360 to check whether the silica concentration in the brine pre-treated at block 362 is above the predetermined threshold (thresh22). The blocks 360 and 362 may be performed iteratively until the silica concentration in the brine are at or below the predetermined threshold (thresh22). Where the brine includes silica concentration that is at or below the predetermined threshold (thresh22), the chemical pre-treatment processing stage is bypassed and the brine pre-treatment process 300 proceeds to block 364.

As shown in **Figure 3C,** at block 364, the brine is tested for aluminum (e.g., Al3+ ions) having a concentration greater than a predetermined threshold (thresh23), for instance a 1, 5, 10, or 20 mg/L threshold. Since aluminum is an amphoteric transitional alkali metal element, various forms of aluminum ions are present in the aqueous solution, such as Al3+, AlO2 , Al(OH)3, Al(OH)2+, Al(OH)2+, which makes the removal of Al more difficult. Methods for removing aluminum from liquids all require the addition of acid, base or extractant, which will have a certain impact on subsequent processes.

As shown in **Figure 3C,** where the brine includes concentration of aluminum that exceeds the predetermined threshold (threshold23), a chemical treatment, chemical precipitation, membrane separation, nanofiltration, ultrafiltration, and/or solvent extraction processing stage is performed on the brine at block 366.

After the chemical treatment, chemical precipitation, membrane separation, nanofiltration, ultrafiltration, and/or solvent extraction of the brine at block 366, the pre-treatment process 300 may return to the block 264 to check whether the aluminum concentration in the brine pre-treated at block 366 is above the predetermined threshold (thresh23). The blocks 364 and 369 may be performed iteratively until the aluminum concentration in the brine is at or below the predetermined threshold (thresh23). Where the brine includes aluminum concentration that is at or below the predetermined threshold (thresh23), the chemical treatment, chemical precipitation, membrane separation, nanofiltration, ultrafiltration, and/or solvent extraction processing stage is bypassed and the brine pre-treatment process 300 proceeds to block 368.

As shown in **Figure 3C,** at block 368, the brine is tested for iron ($Fe^{2+}$) having a concentration greater than a predetermined threshold (thresh24), for instance a 1, 5, or 10 mg/L threshold or manganese ($Mn^{2+}$) having a concentration greater than a predetermined threshold (thresh25), for instance a 10, 50, 100 mg/L threshold. The presence of iron or manganese in the brine may pose challenges similar to those of silica in the brine. Therefore, pre-treatment of the brine to reduce the iron and/or manganese concentration in the brine may improve the efficiency and reliability of DLE and help to minimize harm to ecosystems and ensuring compliance with environmental regulations.

As shown in **Figure 3C,** where the brine includes iron concentration that exceeds the predetermined threshold (thresh24) or manganese concentration that exceeds the predetermined threshold (thresh25), a chemical treatment, ion exchange, and/or membrane separation processing stage is performed on the brine at block 370.

In some aspects, the chemical treatment at block 370 is a coagulation chemical treatment, a flocculation chemical treatment, or a combination of thereof.

After the chemical treatment, ion exchange, and/or membrane separation of the brine at block 370, the pre-treatment process 300 may return to the block 368 to check whether the iron concentration in the brine pretreated at block 368 exceeds the predetermined threshold (thresh24) or the manganese concentration exceeds the predetermined threshold (thresh25). The blocks 368 and 370 may be performed iteratively until the iron concentration is at or below the predetermined threshold (thresh24) and the manganese concentration is at or below the predetermined threshold (thresh25). Where the brine includes the iron concentration at or below the predetermined threshold (thresh24) and the manganese concentration at or below the predetermined threshold (thresh25), the chemical treatment, ion exchange, and/or membrane separation processing stage is bypassed and the brine pre-treatment process 300 proceeds to block 372.

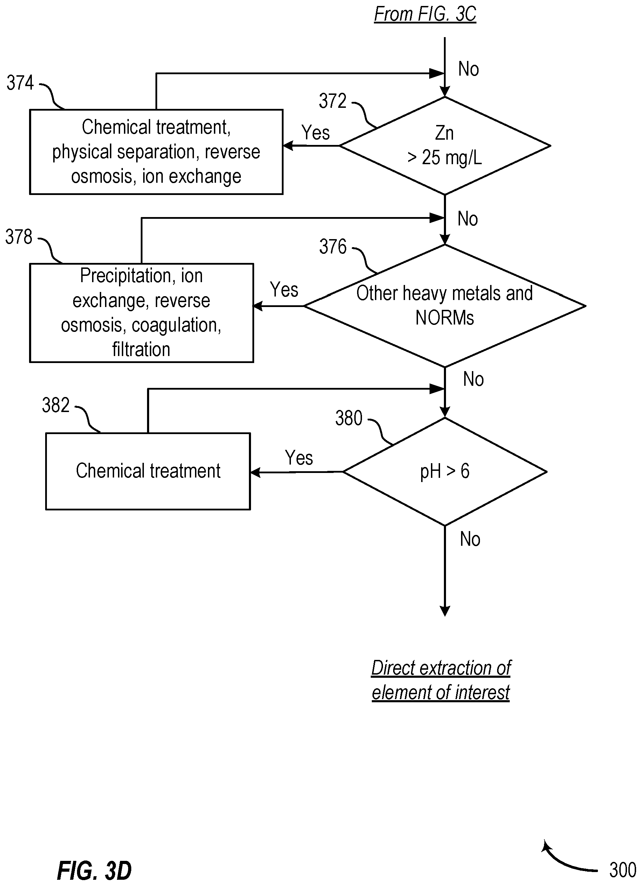

As shown in **Figure 3D,** at block 372, the brine is tested for zinc (Zn) having a concentration greater than a threshold (thresh26), for instance a 10, 25, or 50 mg/L threshold. The presence of zinc in the brine may pose challenges similar to those of silica, iron, or manganese in the brine. Therefore, pre-treatment of the brine to reduce the zinc concentration may improve the efficiency and reliability of lithium extraction and help to minimum harm to ecosystems and meet environmental regulations.

As shown in **Figure 3D,** where the brine includes zinc concentration that exceeds the predetermined threshold (thresh26), a physical separation, chemical treatment, reverse osmosis, and/or ion exchange pre-treatment processing stage is performed on the brine at block 374.

In some aspects, the chemical pre-pretreatment at block 374 is a coagulation chemical pre-treatment, a flocculation chemical pre-treatment, or a combination of thereof.

After the physical separation, chemical treatment, reverse osmosis, and/or ion exchange pre-treatment of the brine at block 374, the pre-treatment process 300 may return to the block 372 to check whether the zinc concentration in the brine pretreated at stage 374 is above the predetermined threshold (thresh26). The blocks 372 and 374 may be performed iteratively until the zinc concentration in the brine is at or below the predetermined threshold (thresh26). Where the brine includes zinc concentration that is at or below the predetermined threshold (thresh26), the physical separation, chemical treatment, reverse osmosis, and/or ion exchange pre-treatment processing stage is bypassed and the brine pre-treatment process 300 proceeds to block 376.

As shown in **Figure 3D,** at block 376, the brine is tested for the presence of other heavy metals (i.e., heavy metals besides silica, Fe, Zn, and Mn) and naturally occurring radioactive materials (NORMs). The presence of heavy metals in the brine may pose challenges similar to those of silica, iron, manganese, or zinc. The other heavy metals may include nickel (Ni), copper (Cu), arsenic (As), cadmium (Cd), hafnium (Hf), and lead (Pb). NORMs may include radium (e.g., $^{226}Ra$, $^{228}Ra$), uranium (Ur), and radon (Rn). Therefore, pre-treatment of the brine to reduce the presence of heavy metals and NORMs may improve the efficiency and reliability of lithium extraction and help to minimum harm to ecosystems and meet environmental regulations.

In some aspects, the concentration of metals in the brine, such as iron, magnesium, zinc, and other heavy metals, is measured using a colorimetry technique.

In some aspects, the concentration of metals in the brine, such as iron, magnesium, zinc, and other heavy metals, is measured using a spectroscopy technique. For example, the concentration of metals in the brine, such as silica, iron, magnesium, zinc, and other heavy metals, may be measured using an atomic absorption spectroscopy (AAS) technique involves analyzing the absorption of light by iron atoms at a specific wavelength, an inductively coupled plasma optical emission spectroscopy (ICP-OES) technique, or an ICP mass spectrometry technique.

In some aspects, the presence of NORMs in the brine may be measured using a gamma spectrometry technique that involves using a gamma-ray spectrometer to detect and analyze gamma radiation emitted by radioactive isotopes in the brine. Each radioactive isotope emits gamma rays at specific energies, allowing for the identification and quantification of the types and amounts of NORMs present.

In some aspects, the presence of NORMs in the brine may be measured using a scintillation counting technique that involves using scintillation detectors to measure gamma radiation emitted by NORMs. These detectors contain materials that emit flashes of light (scintillations) when they interact with gamma radiation. The intensity of the scintillations is proportional to the amount of radiation and can be used to quantify NORM concentrations.

In some aspects, the presence of NORMs in the brine may be measured using an alpha spectrometry technique. Alpha radiation is emitted by some NORMs, such as radium and thorium isotopes. Alpha spectrometry involves using alpha detectors to measure the energy spectrum of alpha particles emitted by these isotopes to identify and quantify the alpha-emitting NORMs.

In some aspects, the presence of NORMs in the brine may be measured using a neutron activation analysis technique that involves exposing the brine sample to a neutron source, which can activate certain elements in the sample. The resulting radioactive isotopes can then be quantified using gamma spectrometry or other detection methods.

In some aspects, the presence of NORMs in the brine may be measured using an ICP-MS technique.

As shown in **Figure 3D,** where the brine includes other heavy metals or NORMs, a precipitation, ion exchange, reverse osmosis (RO), coagulation, filtration, and/or electrodialysis pre-treatment processing stage is performed on the brine at block 378. RO may be used by remove the heavy metals and/or NORMs from the brine by pressurizing the brine using a high-pressure pump to force the brine though an RO membrane. The pressure applied may be higher than the osmotic pressure of the brine, which allows the brine to pass through the semi-permeable RO membrane while retaining the impurities. The RO membranes have very fine pores that selectively allow certain molecules to pass through while rejecting larger ions, molecules, and contaminants.

After the precipitation, ion exchange, RO, coagulation, filtration, and/or electrodialysis pre-treatment of the brine at block 378, the pre-treatment process 300 may return to the block 376 to check whether the other heavy metals or NORMs are present in the brine pretreated at block 378. The blocks 376 and 378 may be performed iteratively until the brine does not contain presence of the other heavy metals or NORMs. Where the brine does not have the other heavy metals or NORMs present, the precipitation, ion exchange, RO, coagulation, filtration, and/or electrodialysis pre-treatment processing stage is bypassed and the brine pre-treatment process 300 proceeds to block 380.

As shown in **Figure 3D,** at block 380, the brine is tested for a pH level above a threshold (thresh27), for instance a 5.5, 6, or 6.5 threshold. A high pH level brine may cause lithium compounds to precipitate or form solid particles. For example, lithium carbonate ($Li_2CO_3$) can precipitate from brine at elevated pH levels. This precipitation can lead to the loss of valuable lithium from the brine, reducing the lithium recovery efficiency. Further, high pH conditions can promote the formation of scale deposits and fouling in processing equipment. Further, high-pH brines may contain elevated concentrations of alkaline impurities, such as hydroxides and carbonates. These impurities can interfere with the efficiency of lithium extraction methods, including ion exchange, solvent extraction, or adsorption processes. In addition, some lithium extraction processes involve chemical reactions that are sensitive to pH levels. A high pH level can alter the kinetics and thermodynamics of these reactions, potentially reducing their effectiveness. Further, elevated pH levels can increase the solubility of certain undesirable ions present in the brine, such as magnesium and calcium. These ions can co-extract with lithium, affecting the purity of the final lithium product. Moreover, discharging high-pH brine into the environment can have negative environmental impacts, particularly in aquatic ecosystems. High-pH brines can disrupt the pH balance of receiving waters, potentially harming aquatic life. Therefore, pre-treatment of the brine to reduce the pH level may improve the efficiency and reliability of DLE and help to minimize harm to ecosystems and ensure compliance with environmental regulations.

In some aspects, the pH level of the brine is measured using a pH meter or pH indicator strips.

As shown in **Figure 3C,** where the brine includes a pH level that exceeds the predetermined threshold (thresh27), a chemical pre-treatment processing stage is performed on the brine at block 382. The chemical pre-treatment lowers the pH level of the brine by adding acidic substances to the brine, such as hydrochloric acid (HCl), sulfuric acid ($H_2SO_4$), carbon dioxide ($CO_2$), acetic acid ($CH_3COOH$), citric acid ($C_6H_8O_7$), or phosphoric acid ($H_3PO_4$) and/or by adding buffering agents (e.g., sodium bisulfite ($NaHSO_3$) or sodium carbonate).

After the chemical pre-treatment of the brine at block 382, the pre-treatment process 300 may return to the block 380 to check whether the pH level in the brine pretreated at stage 382 is below the desired threshold (thresh27). The blocks 380 and 382 may be performed iteratively until the pH level in the brine is at or below the pH predetermined threshold (thresh27). Where the brine pH level is at or below the predetermined threshold (thresh27), the chemical pre-treatment processing stage is bypassed and the brine pre-treatment process 300 completes and the brine may be further processed for lithium extraction (e.g., as described with respect to **Figures 1-2),** for example, the brine may proceed directly to an extraction stage or may proceed to an extraction stage by passing first through intermediate stages, such as pre-concentration.

While **Figures 3A-3D,** illustrate a particular sequence of different brine pre-treatment stages designed to remove particular impurities, and/or change one or more properties of the brine, harmful to the direct extraction from the brine based on particular predetermined ranges/thresholds, different combinations or subsets and different ordering of brine pre-treatment stages may be used and may remove different impurities and/or be based on different predetermined ranges/thresholds. According to certain aspects, the multiple brine pre-treatment stages may generally be ordered from coarser to finer granularity of brine pre-treatments.

In some aspects, the respective brine pre-treatment are performed by routing the brine to different equipment, which may be collocated or at different locations.

In some aspects, different brines extracted from different geological locations may need different amounts of brine pre-treatment before the brine suitable for DLE processing. Accordingly, in some aspects, the measurements of the brine impurities and the routing to or bypassing of the various brine pre-treatment stages are done dynamically in real-time.

In some aspects, one or more initial samples of brine are tested during a pre-planning/design/construction phase to determine an initial set of brine pre-treatment stages. In such cases, a resulting extraction flowsheet may be designed taking into account the pre-treatment stages. For instance, the method may include selecting the stage(s) and related equipment of the extraction flowsheet to not include in the flowsheet a pretreatment stage that has been decided to be bypassed based on the representative sample or, if several representative samples are involved, based on the majority or all of the samples. Alternatively, the method may include selecting the pre-treatment stages and related equipment of the extraction flowsheet to include a brine pretreatment stage(s) in the flowsheet that has not been bypassed based on the representative sample or, if several representative samples are involved, based on the majority or all of the them. Furthermore, the method may include selecting the stage(s) and related equipment of the extraction flowsheet in the flowsheet to include a predetermined number of instances of a pretreatment stage if the representative sample or, if several representative samples are involved, the majority or all of the representative samples, have gone through said stage said predetermined number of times.

In some aspects, during operation, the pre-treatment process is dynamically updated.

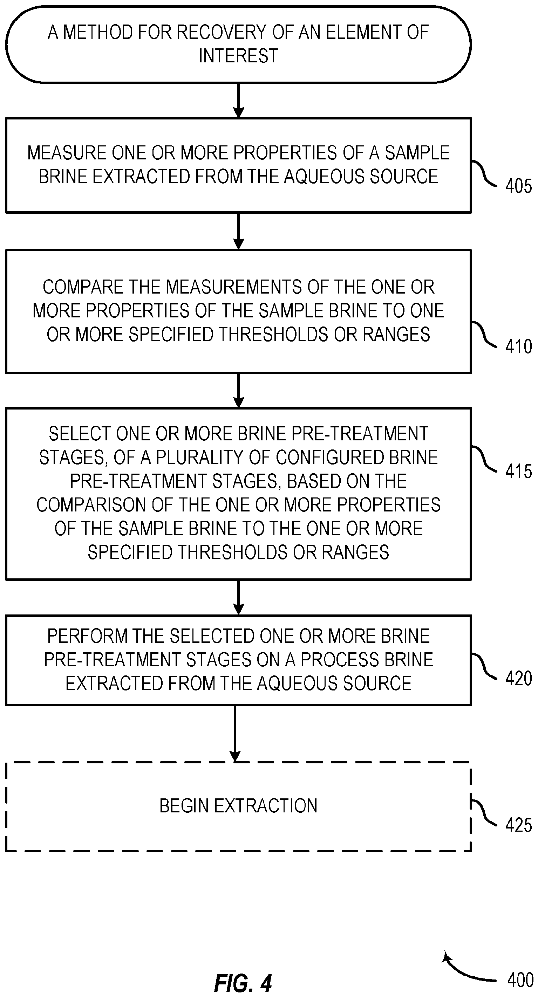

**Figure 4** depicts a process flow 400 for brine pretreatment that can be processed before recovering lithium or other elements of interest from an aqueous source according to certain aspects. In some aspects, the process flow 400 may be an example of the brine pre-treatment and DLE processes depicted and described with respect to **Figures 1-3.**

The process flow 400 includes, at operation 405, measuring one or more properties of a sample brine extracted from an aqueous source.

In some aspects, the process flow 400 further includes, at operation 410, comparing the measurements of the one or more properties of brine to one or more specified ranges/thresholds.

In some aspects, the process flow 400 further includes, at operation 415, selecting one or more brine pre-treatment stages, of a plurality of configured brine pre-treatment stages, based on the comparison of the one or more properties of the sample brine to the one or more specified ranges/thresholds.

In some aspects, the process flow 400 further includes, at operation 420, performing the selected one or more brine pre-treatment stages on a process brine extracted from the aqueous source.

In some aspects, the one or more properties of the brine comprises a concentration of lithium in the brine; the one or more ranges/thresholds comprises a minimum lithium concentration threshold and a maximum lithium concentration threshold; and the process flow 400 further comprises: not processing the brine when the concentration of the lithium in the brine is smaller than a minimum lithium concentration threshold; bypassing the plurality of brine pre-treatment stages when the concentration of the lithium in the brine is larger than a maximum lithium concentration threshold; and proceeding to a first brine pre-treatment stage when the concentration of the lithium in the brine is between the minimum and maximum lithium concentration thresholds.

In some aspects, selecting at operation 415 the one or more brine pre-treatment stages, of the plurality of configured brine pre-treatment stages, comprises: evaluating brine pre-treatment stages, of the plurality of configured brine pre-treatment stages, in a preconfigured order.

In some aspects, successive brine pre-treatment stages, of the plurality of configured brine pre-treatment stages, modifies one or more properties of the process brine at a finer granularity than earlier brine pre-treatment stages of the plurality of brine pre-treatment stages.

In some aspects, the one or more properties of the brine includes concentration of total suspended solids (TSS), concentration of oil droplets, concentration of grease, concentration of dissolved gases, concentration of dispersed hydrocarbons, organic acids or other water soluble organics, concentration of microorganisms and their metabolite byproducts, concentration of production chemicals, concentration of silica, concentration of iron, concentration of manganese, concentration of other divalent and trivalent metals, concentration of heavy metals, concentration of naturally occurring radioactive materials (NORMs), concentration of anions, a pH level, a COD level, a BOD level, a TOC level, concentration of other deleterious elements, or a combination thereof.

In some aspects, the plurality of brine pre-treatment stages includes a plurality of chemical pre-treatment, precipitation pre-treatment, advanced oxidation pretreatment, ion exchange pre-treatment, adsorption pre-treatment, absorption pre-treatment, activated carbon pre-treatment, membrane separation pre-treatment, forward and reverse osmosis pre-treatment, physical separation pre-treatment, gravity separation pre-treatment, American petroleum institute (API) separator, filtration pre-treatment, microfiltration pretreatment, nanofiltration pre-treatment, ultrafiltration pre-treatment, plate pack interceptor pre-treatment, pre-treatment sieve pre-treatment, skim tank pre-treatment, hydrocyclone pre-treatment, centrifuge pre-treatment, coagulation pre-treatment, flocculation pretreatment, gas flotation pre-treatment, gas purging pre-treatment, biological pre-treatment, chemical disinfection pre-treatment, ultraviolet (UV) radiation pre-treatment, electrochemical pre-treatment, electrodialysis pre-treatment, or a combination thereof.

In some aspects, evaluating the brine pre-treatment stages, of the plurality of configured brine pre-treatment stages, in the preconfigured order comprises for each respective brine pre-treatment stage: comparing a measurement of a one or more properties of the brine to a one or more specified ranges/thresholds; when the measurement of the specified property of the brine is within a specified range/threshold: bypassing the respective brine pre-treatment stage; and proceeding to a next brine pre-treatment stage of the plurality of brine pre-treatment stages; and when the measurement of the specified property of the brine is outside the specified maximum range/threshold: performing the respective brine pre-treatment stage; and then proceeding to the next brine pre-treatment stage of the plurality of brine pre-treatment stages.

In some aspects, multiple brine pre-treatment stages of the plurality of brine pre-treatment stages are associated with a same property of the brine, and wherein the multiple brine pre-treatment stages associated with the same property of the brine are associated with different ones of the one or more specified ranges/thresholds.

In some aspects, the process flow 400 further comprising computing a quantitative score of a brine pre-treatment process based on the selected one or more brine pre-treatment stages of the plurality of configured brine pre-treatment stages.

In some aspects, the performing the selected one or more brine pre-treatment stages on the brine at operation 420 is prior to beginning lithium extraction at operation 425.

Note that process flow 400 is just one example of a method, and other methods including fewer, additional, or alternative steps are possible consistent with this disclosure.

### Quantitative Brine Analysis for Mineral Extraction

Methods for quickly assessing the quality of brines are desirable in order to determine whether a brine meets composition requirements for the critical mineral extraction processes, for example, to meet a target extraction yield. Such assessment may help with selecting the extraction technologies and sizing of those extraction technologies in order to maximize the mineral recovery and system reliability, while minimizing processing time and costs. Further, brines may be produced as byproducts, and these brines may be assessed to prevent corrosion, clogging, for environmental impact, and/or sometimes for reuse of the brines to enhance oil recovery.

Aspects of the present disclosure provide a workflow for simple, fast, and consistent and yet quantitative assessment of brines for various purposes such as economic viability assessment and/or operational feasibility study of brines for critical minerals extraction. The workflow may be used by investors and/or operators to design, plan, and execute mineral extraction projects profitably.

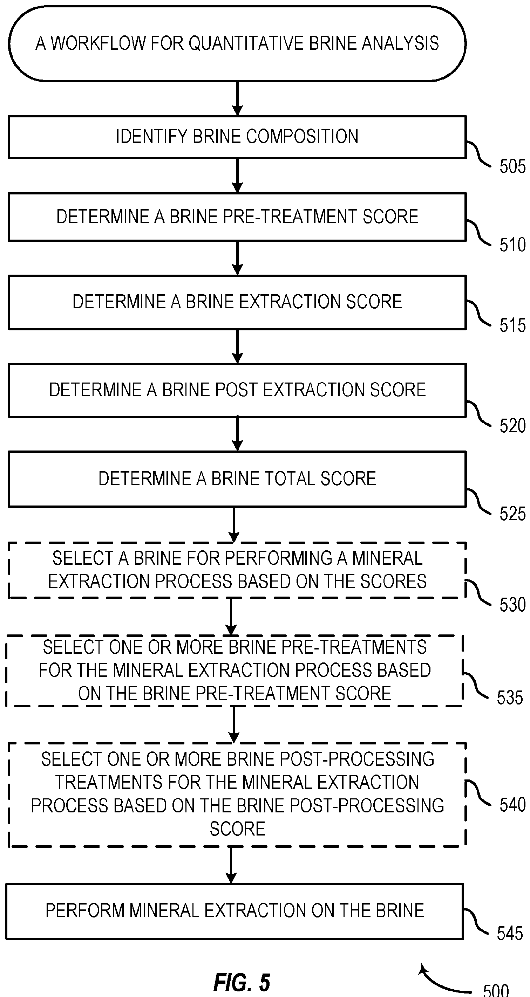

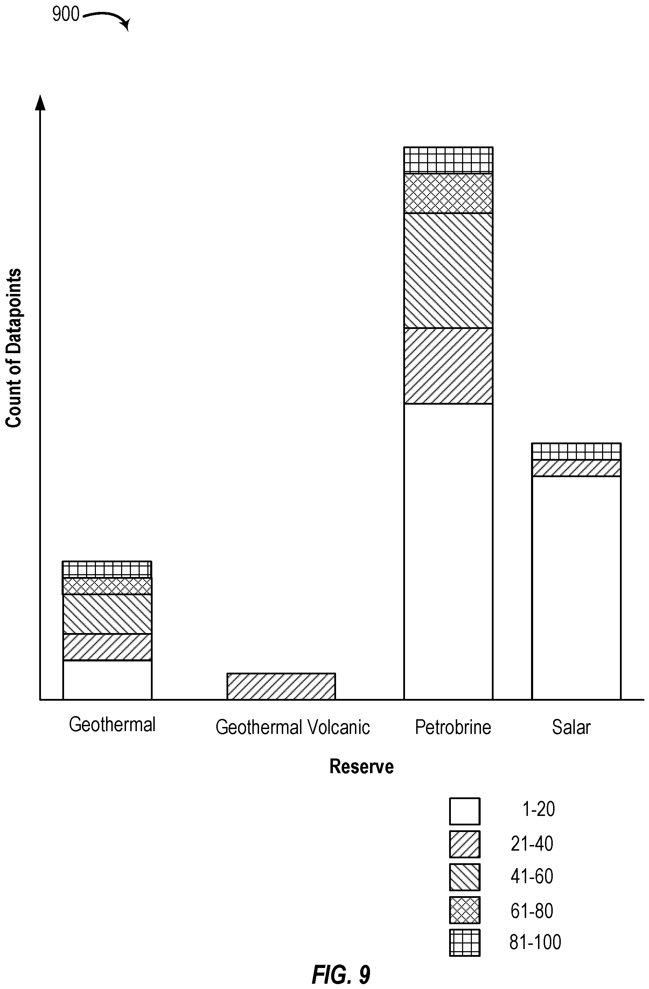

**Figure 5** illustrates an example workflow 500 for the quantitative brine analysis. Aspects of the workflow 500 may be performed by a system for quantitative brine analysis, such as, for example, the system 900 discussed in more detail below with respect to **Figure 9.**

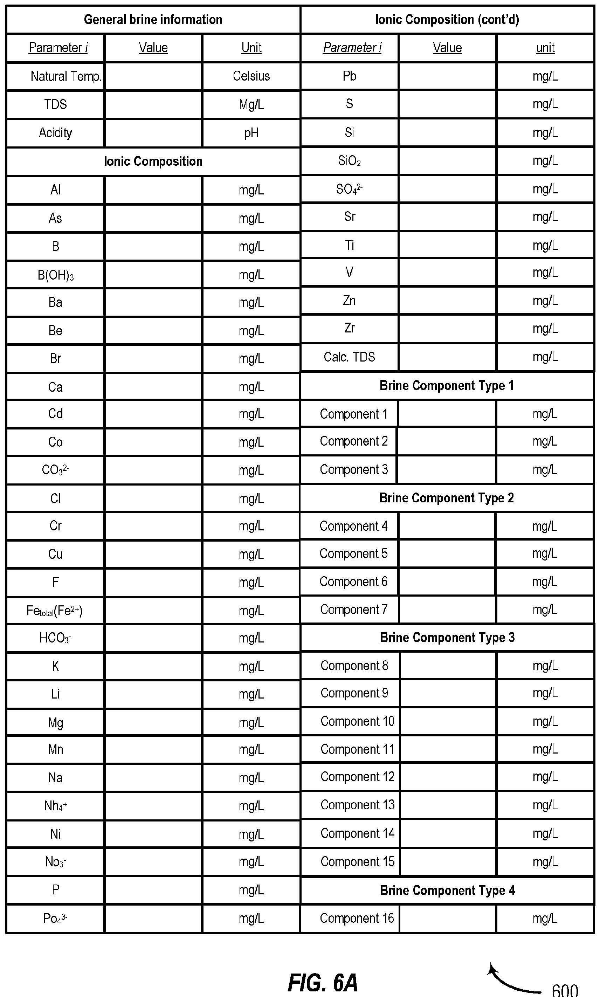

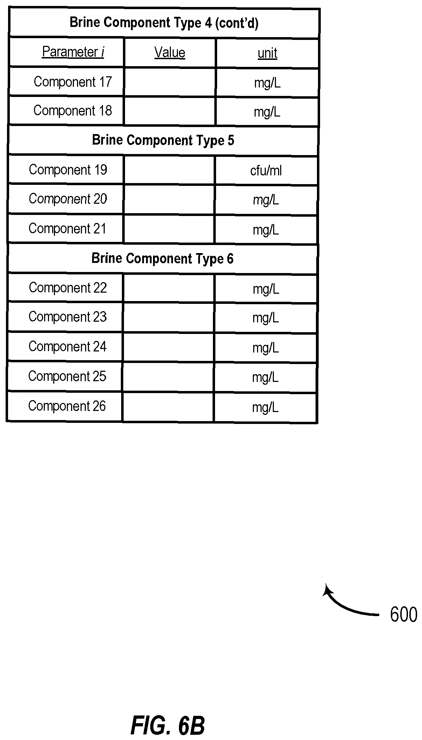

In some aspects, the workflow 500 may begin, at operation 505, with identifying a composition of the brine. For example, **Figures 6A-6B** depict a table 600 illustrating example information that may identified for the brine. As shown, operation 505 may include identifying multiple parameters of the brines, which include physicochemical parameters associated with the brine. For example, the parameters may include general brine information parameters, such as a natural temperature parameter, a total dissolved solids (TDS) parameter, and an acidity (pH) of the brine parameter. As shown, the parameters may also include parameters corresponding to the chemical analysis of brine, where the concentration of each ion is treated as an ionic composition parameter, and a number of additional brine composition type parameters (e.g. components 1-26 for the additional brine component types 1-6) corresponding to the concentrations of additional types of materials in the brine (e.g., any of impurity identified herein that may impact a mineral extraction process). Ions may include any of the ions shown in the table 600, or any other ion of interest. In some aspects, the additional brine component types include neutral species parameters, solids parameters, hydrocarbon parameters (e.g., oil, grease, dispersed hydrocarbons, and/or dissolved hydrocarbons), organics parameters (e.g., organic acids), dissolved gases parameters, production chemicals parameters, and bacteria parameters (e.g., bacteria cell count or biochemical oxygen demand (BOD).

In some aspects, the composition of the brine may be identified using any approach, technology, tools, and/or sensors for identification of component concentrations in the brine. In some aspects, the composition of the brine (e.g., the input shown in table 600) may be input to the system for quantitative brine assessment. In some aspects, the composition of the brine may be entered manually. In some aspects, the composition of the brine may be identified and automatically provided to the system, by the brine composition equipment and/or sensors, for quantitative brine assessment without requiring user input.

The input table provided in **Figures 6A-6B** is only exemplary and not limiting the scope of the invention. All or part of the information of this table may be used in the quantitative brine assessment, potentially along other information derived from brine composition analysis. While certain general brine information parameters, ionic composition parameters, and the six additional brine component types 1-6 are illustrated in the table 600 with various numbers of components (i.e., components 1-26) for each of the brine component types, it should be understood that these examples are illustrative, and that different brine information, different numbers of brine composition types, different parameters, different numbers of parameters, different components, and different numbers of components, may identified as input for the quantitative brine analysis described herein.

Referring again to **Figure 5,** as shown, the workflow 500 may proceed, at operation 510, with determining a brine pre-treatment score. In some aspects, brine pre-treatment steps are evaluated prior to the mineral extraction process. The brine pre-treatment score may be calculated based on the concentration of brine parameters that are harmful to the sorbent materials in the extraction process. For example, the concentration of such harmful brine parameters may involve additional pre-treatment stages, as described above with respect to **Figures 2-4,** to remove those parameters from the brine before the mineral extraction.

In some aspects, a pre-treatment score (*PTS*) subscore can be computed as:

$$PTS = 100 \text{ X } \left(1 - \frac{\sum_{i=1}^{n} S_i W_i P_i}{\sum_{i=1}^{n} W_i}\right),$$

where the total number of parameters *i* denoted by *n.* For example, referring to the table 600, n is the total number of the parameters.

The *PTS* is over the summation of the parameters, *i=1...n,* an associated weight $W_i$, and an associated function $P_i$ Each parameter *i* may be assigned a coefficient $S_i$ having a value of either 0 or 1, indicating whether the parameter *i* is harmful to the sorbent or not (e.g., and whether a pre-processing stage for that parameter is required or not). As discussed in more detail below, the coefficient value ($S_i$) for each parameter may be different for the different subscores based on whether a given parameter has an impact on the particular process associated with the respective subscores. For example, the coefficient value is $S_i$ for the *PTS*, $S_i'$ for the extraction score, and $S_i''$ for the post-extraction score. As an illustrative example, the parameter "Lithium" may have an impact on the mineral extraction process (Extraction subscore) but not on the brine pre-treatment process (Pretreatment subscore) and, in that case, $S_{Li}$ = 0 and $S_{Li}'$ = 1. Depending on the sorbent type, some parameters (e.g., silica, iron, and $H_2S$) may be harmful to the sorbent (resulting in S; = 1 and necessitating pretreatment), while other parameters may not be harmful to the sorbent (resulting in $S_i$ = 0 and no pre-treatment required). In some aspects, $S_i$ may be determined by comparing each parameter of the brine sample to at least one specific threshold (for instance, if silica concentration is over 5 mg/L, then $S_i$ = 1; otherwise $S_i$ = 0).

The associated weight $W_i$ for a parameter may be associated with various factors associated with the cost or difficulty in mitigating that parameter, for example, to change the value of that parameter to an acceptable level. For example, the associated weight $W_i$ for a parameter can be based on the process efficiency (e.g., extraction process yield, product impurity grade), energy consumption, capital cost (e.g., equipment cost, lead time), operating cost (e.g., utilities costs, labor costs, a cost of pre-treatment stage for the parameter), logistics, technical feasibility (e.g., technology accessibility), environmental impact, environment regulations, and/or other applicable regulations. The value of the associated weight $W_i$ for each parameter may be different (e.g., although determined or specified based on the same considerations) and may be different for the different subscores based on the considerations of the particular process associated with the respective subscores. For example, the value of the associated weight for a given parameter *i* may be $W_i$ for the *PTS*, $W_i'$ for the extraction score, and $W_i''$ for the post-extraction score.

$P_i$ is a function representing how impactful the parameter is on the mineral pre-treatment process. In some aspects, $P_i$ can be any non-zero empirical equation, including 1 or a constant. In some aspects, where $P_i$ only depends on presence of the parameter *i*, $P_i$ is a constant. In some aspects, $P_i$ may account for a value or amount $Y_i$ of the parameter *i*, such as the concentration, temperature, TDS, or level of acidity. In this case, *ES* may be calculated as a function of $Y_i$ as follows:

$$P_i = \frac{1}{1+\exp\left[k_{1i}(log Y_i - k_{2i})\right]},$$

where $k_{1i}$, and $k_{2i}$ are empirical coefficients, and their values may be different for different parameters. $P_i$ may be different for the different subscores based on the considerations of the particular process associated with the respective subscores. For example, $P_i$ for a given parameter $i$ may be $P_i$ for the *PTS, $P_i'$* for the extraction score, and $P_i''$ for the post-extraction score.

The *PTS* subscore provides a quantitative analysis of the brine pre-treatment process, indicating the quality and suitability of the process brine supplied from an aqueous source for the direct extraction process. The *PTS* evaluates the physiochemical characteristics and the presence of the impurities in the brine and the required pretreatment processes for those impurities. Depending on the brine composition, some brines may require only a few pre-treatment steps to be ready for mineral extraction, while other brines may require more extensive pre-treatment before initiating mineral extraction. A higher PTS score may correspond to a lower number of required pre-treatment steps and a higher quality brine, while a lower *PTS* score may correspond to a higher number of required pre-treatment steps and a lower quality brine. Thus, the pre-treatment score is a measure of the effectiveness of the pretreatment process. In some aspects, a *PTS* score of zero may indicate that the brine must undergo all available pre-treatment stages before the mineral extraction process for the brine, while a *PTS* of 100 may indicate that no pre-treatment is needed for the brine.

As shown in **Figure 5,** the workflow 500 may proceed to operation 515, with determining an extraction score (*ES*) for the brine. The extraction score may be calculated based on the values of some specific parameters of brine that directly impact the yield and recovery of sorbent-based extraction processes, for example from the identified composition information in table 600.

In some aspects, an extraction score (*ES*) subscore can be computed as:

$$ES = k \prod_{i=1}^{n} \left(\frac{C_{Mi}}{C_E}\right)^{\alpha i},$$

where, $C_E$ is concentration of element of interest $E$ in the brine, $C_{Mi}$ is concentration of impurity $M_i$ in the brine, $\alpha_i$ is an empirical exponent corresponding to the $M_i$ impurity, and $k$ is a scaling factor. Concentrations may be in mg/L and $\alpha_i$ exponents may be positive or negative depending on the effect of $M_i$ on the mineral extraction efficiency and yield. The extraction score provides a quantitative analysis of the element of interest extracted from the brine compared to impurities. For example, in the case of extraction of the element of interest $E$ in the presence four impurities, the extraction score equation can be expanded as follows:

$$ES = k \left(\frac{M_1}{E}\right)^{\alpha_1} \left(\frac{M_2}{E}\right)^{\alpha_2} \left(\frac{M_3}{E}\right)^{\alpha_3} \left(\frac{M_4}{E}\right)^{\alpha_4}$$

In this example, $\alpha_1$ may have a positive value and $\alpha_2$, $\alpha_3$, $\alpha_4$ may have negative values. A higher *ES* may indicate that the brine is better suited for the desired extraction process.

In some aspects, the *ES* subscore can be computed as:

$$ES = 100 \text{ X} \left(\frac{\sum_{i=1}^{n} S_i' W_i' P_i'}{\sum_{i=1}^{n} W_i'}\right).$$

Similar to above, $S_i'$ is a coefficient for each parameter set based on whether that parameter impacts the mineral extraction. $S_i'$ is set to 1 if the parameter affects the mineral extraction stage; otherwise, $S_i'$ is set to 0. The weight $W_i'$ is an indicator of the impact or importance of the parameter relative to the extraction stage. $P_i'$ is a function representing how impactful the parameter $i$ is on the extraction process, which may account for the amount $Y_i$ of the parameter present, such as the concentration, temperature, TDS, or level of acidity.

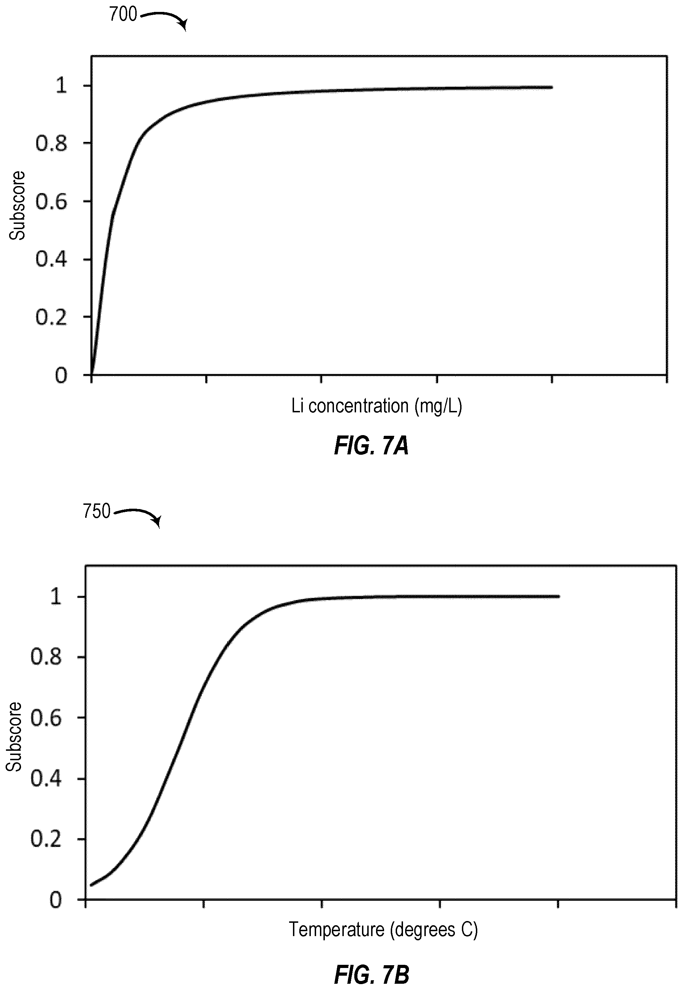

In an illustrative example, **Figure 7A** is a graph 700 depicting an example *ES* subscore for the parameter Lithium ($i$ = Li), where $S_{Li}'$ is 1 and $P_{Li}'$ is a function of the concentration of Li in the brine ($Y_{Li}$ = mg/L Li). **Figure 7B** is a graph 750 depicting an example *ES* subscore for the parameter temperature of the brine ($i$ = temperature), where $S_{temp}'$ is 1 and $P_{temp}'$ is a function of the temperature value ($Y_{temp}$ = degrees C).

Referring again to **Figure 5,** as shown, the workflow 500 may proceed, at operation 520, with determining a brine post extraction score (PES), or post-processing, subscore. Post-processing may include processing steps performed post sorbent-based extraction. One example of a post processing step is reverse osmosis membrane separation, such as counter-flow reverse osmosis (CFRO).

The *PES* provides a quantitative analysis of stages of the post-extraction treatment process after the mineral extraction process. In some aspects, a *PES* is calculated based on the number and significance of the parameters that impact the post processing. For example, the *PES* may be calculated based on the concentrations of parameters in the brine that impact the yield and recovery of post sorbent-based extraction processes and the purity of final product. Additionally, the *PES* may be calculated based on post-processing steps required for environmental regulatory compliance. The number of post-processing steps required may depend on the market demand, final product type, the desired purity level, and the stringency of the environmental laws and regulations.

In some aspects, a *PES* can be computed as:

$$PES = 100 \text{ x} \left(1 - \frac{\sum_{i=1}^{n} S_i'' W_i''}{\sum_{i=1}^{n} W_i''}\right).$$

Similar to above, $S_i''$ is a coefficient set to 1 if a parameter that affects the post-extraction stage; otherwise, $S_i''$ is set to 0. The weight value $W_i''$ associated with the parameter $i$ is an indicator of the impact or importance of that parameter relative to the post-extraction stage.

In some aspects, a *PES* can be computed further based on a function $P_i''$:

$$PES = 100 \text{ X } (1 - \frac{\sum_{i-1}^{n} S_i'' W_i'' P_i''}{\sum_{i=1}^{n} W_i''}),$$

where, $P_i'$ may account for the amount $Y_i$ of the parameter in the brine, such as the concentration, temperature, TDS, or level of acidity. A *PES* of 0 may indicate all the parameters exceed harmful levels; conversely, a *PES* of 100 may indicate that all of the defined parameters are below the harmful range.

As shown in **Figure 5,** the workflow 500 may proceed, at operation 525, with determining a brine total score (*TS*). The overall assessment can be done by using customized weighted average of all subscores including the pretreatment, extraction, and post-processing. The total score, ranging from 0 to 100, is a quantitative measure of the brine's quality for critical mineral extraction processes. A score of 100 indicates the highest possible quality of brine for the critical mineral extraction, while a score of 0 indicates the lowest possible quality.

In some aspects, the *TS* can be computed by weighted averaging of the *PTS, ES,* and *PPS/PES,* as:

$$TS = \frac{(PTS \text{ x } W_{PTS}) + (ES \text{ x } W_{ES}) + (PES \text{ x } W_{PES})}{W_{PTS} + W_{ES} + W_{PES}},$$

where $W_{PTS}$ is based on the importance weight of the *PTS* subscore, $W_{ES}$ is based on the importance weight of the *ES* subscore, and $W_{PES}''$ is based on the importance weight of the *PES* subscore. Thus, the total score provides a quantitative analysis of the complete mineral extraction process including the brine pre-treatment process, the extraction process, and the post-extraction treatment.

In an example, taking each of the three scores as having equal importance (the weight parameter equal to 1 for each of subscores), the *TS* equation can be simplified as:

$$TS = \frac{PTS + ES + PES}{3}$$

The proposed quantitative assessment may be based only on chemical composition of brines, without the need to go through the entire critical mineral extraction flowsheet, providing a quick and simple way to obtain a quantitative assessment. In addition, the individual scores from each step can also be used separately to analyze the brine. For example, if two brines have equal total scores, the sub-scores from each step may be used to further analyze the brines.

In some aspects, the total score can be used make decisions on the DLE process. In some aspects, the score can be used to determine whether to a brine pre-treatment stage is needed and/or which brine pre-treatment stages to perform.

As shown in **Figure 5,** the workflow 500 may proceed, at operation 545, with performing a mineral extraction process on the brine based on the brine pre-treatment score, the brine extraction score, the brine post-extraction score, and the brine total score. In some aspects, the workflow 500 includes, at operation 530, selecting a brine for performing the mineral extraction based on the scores. In some aspects, the workflow 500 includes, at operation 535, selecting one or more brine pre-treatments to perform before the mineral extraction based on the brine pre-treatment score. In some aspects, the workflow 500 includes, at operation 540, selecting one or more brine post-processing treatments to perform after, or as part of, the mineral extraction based on the brine post-processing score.

In some aspects, the workflow 500 includes determining an operational feasibility of the brine for mineral extraction. In some aspects, the operational feasibility is determined based on the brine scores, the concentration of the mineral of interest, the TDS, and the acidity of the brine.

In some aspects, the workflow 500 includes mixing brines based on a feasibility of the brines.

In some aspects, the selection of the brine for mineral extraction, the selection of the brine pre-treatments, the selection of brine post-processing, and/or the brine mixing may be selected in order to meet one or more mineral extraction targets or thresholds. The mineral extraction targets or thresholds may include a target yield or threshold, a target or threshold extraction time, a target or threshold profitability, a target or threshold environmental impact, or a combination of targets or thresholds.

In some aspects, the scoring algorithm may be used as a standalone software or integrated into a unified platform along with other software used for equipment sizing (e.g., Aspen HYSYS®, Aspen Plus®, Symmetry™ - a mark of SLB), facility design (e.g., Facility Planner™ - a mark of SLB), and planning and execution (e.g., FDPlan™ - a mark of SLB).

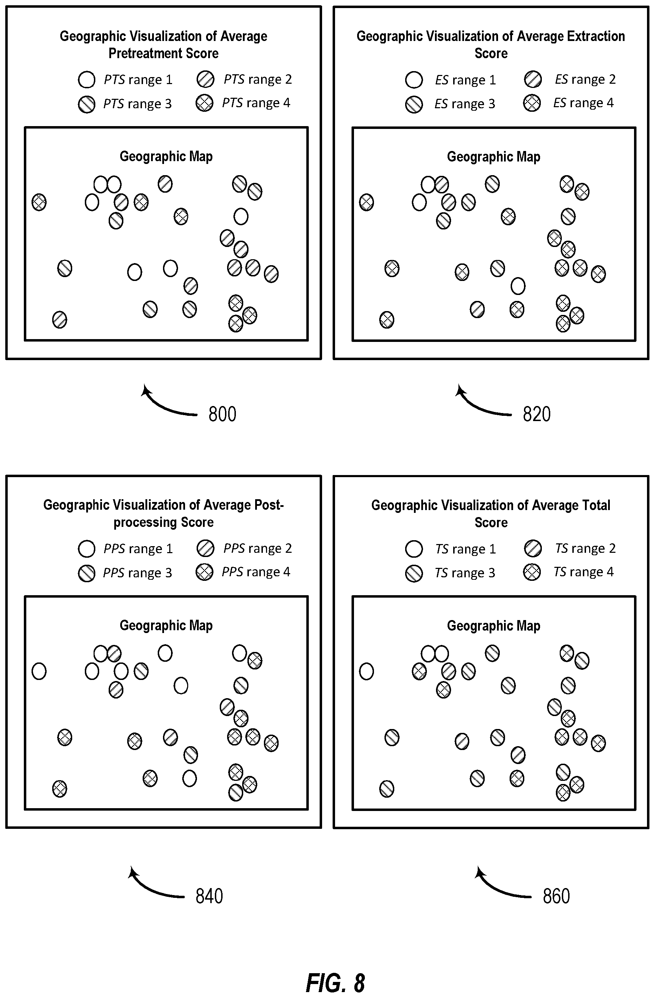

Further, various visualizations of the quantitative brine scoring are proposed to aid in the brine assessment. In some aspects, the scoring algorithm may be implemented to a geochemistry database to assist analyzing the quality of brines based on various filters such as regions, countries, geological formations, reserve type, etc. For example, **Figure 8** depicts a geographic visualization of average pretreatment score 800, a geographic visualization of average extraction score 820, a geographic visualization of average post-processing score 840, and geographic visualization of average total score 860. In some aspects, any combination or all of the visualizations may be displayed to a user. In some aspects, the visualizations may be displayed to the user in separate windows of a display, on separate display screens, or on separate portions of a display window. As shown in **Figure 8,** each visualization may include visualizations of multiple ranges of the respective score, where a score may range in values from 0 to 100, the ranges may include various subranges. In the illustrated example, four subranges are shown, which may correspond to scores of 0-25 (range 1), 26-50 (range 2), 51-75 (range 3), and 75-100 (range 4). It should be understood that while four subranges are illustrated, any number of subranges may be displayed. In some aspects, the subranges may be configurable by the user.

As shown in **Figure 8,** score indicators may be added to a geographic map in each of the visualizations. For example, a score indicator may be added to the geographic map corresponding to the location of an assessed brine associated with the score. In some aspects, the indicator may have a color (or other associated property) indicated the score subrange associated with the assessed brine. As illustrated in **Figure 8,** each circle sown on the geographic map corresponds to a source location of an assessed brine, and the shading of each circle corresponds to one of the score ranges. Thus, by viewing the visualization on a display, a user may be able to easily identify the locations and respective scores (or score range) of brines in the geographic area shown in the map.

In some aspects, the quantitative brine assessment scoring may be coupled with a geochemistry database and can be used for feasibility studies for a specific critical mineral extraction process.

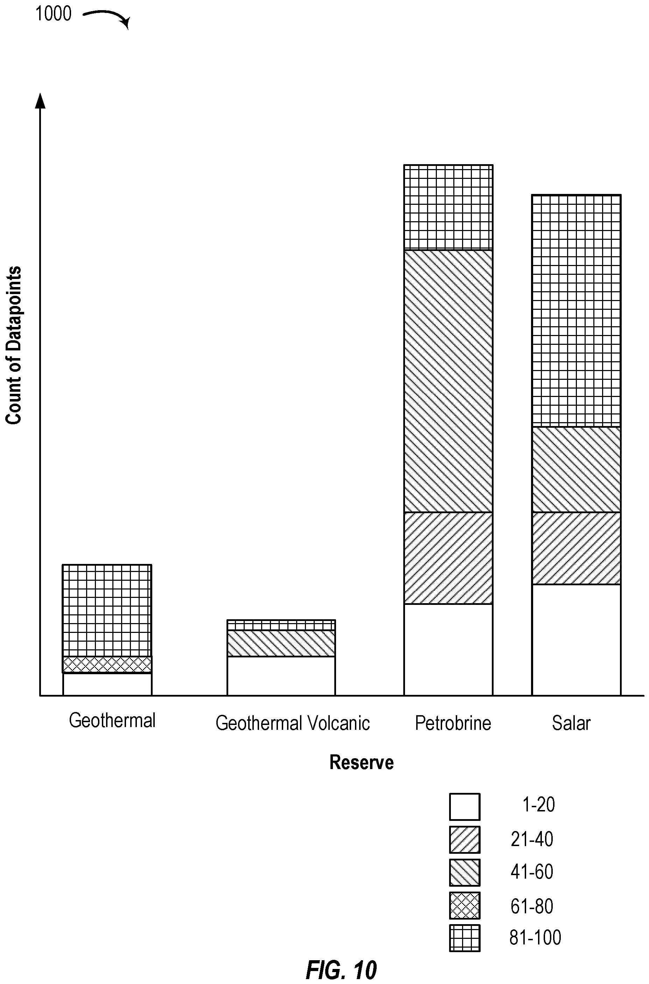

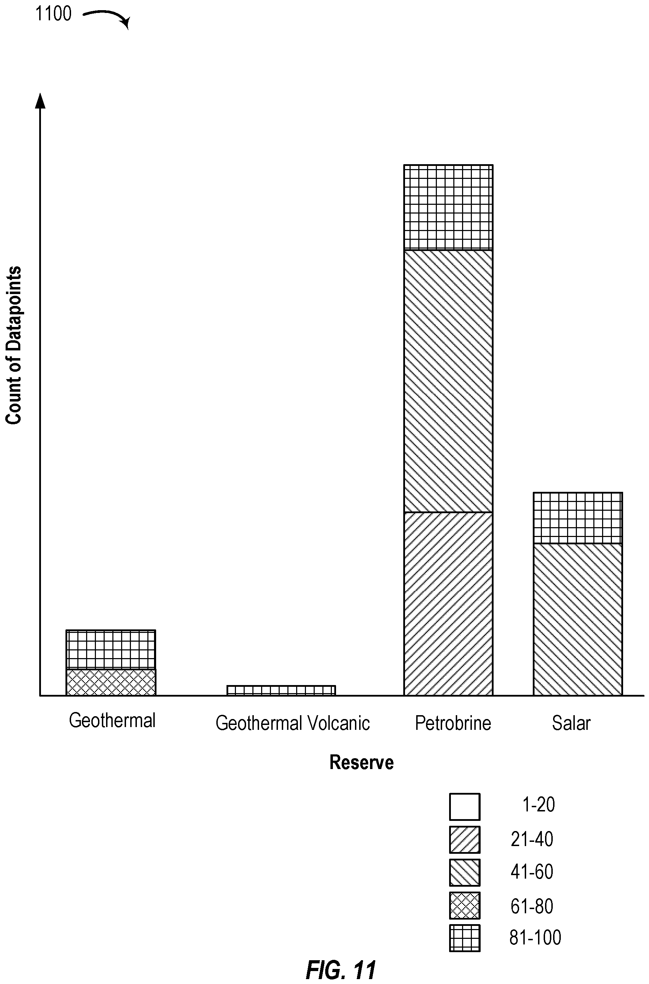

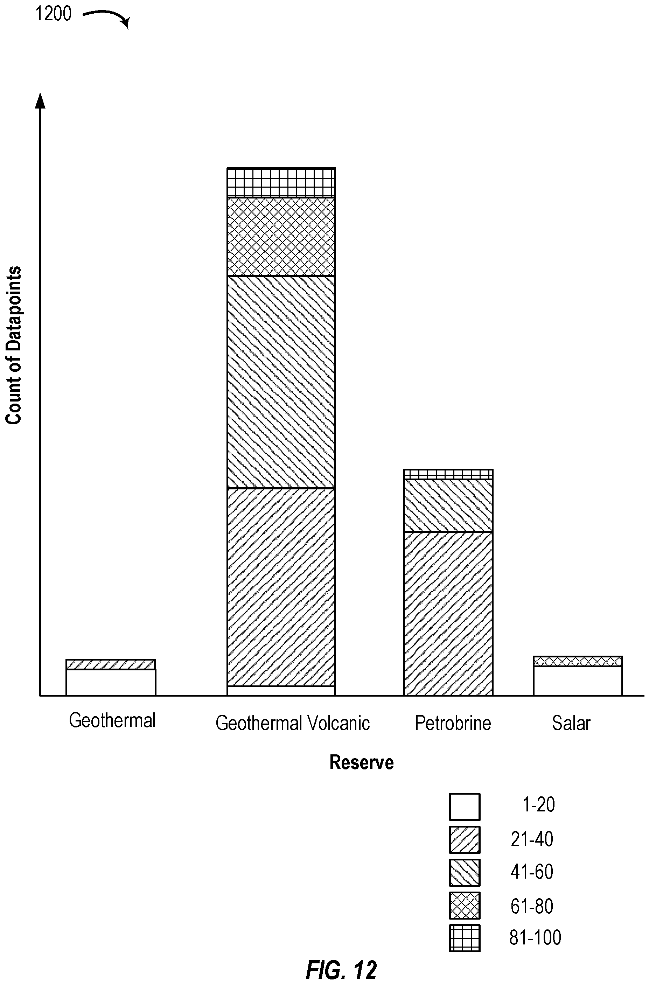

In some aspects, brine assessment score may be stored in a database. In some aspects, counts for the brine scores may be stored and may be associated with a type of the reserve (e.g., geothermal, geothermal volcanic, petrobrine, or salar). In some aspects, the counts may stored with the respective associated score range. **Figure 9** depicts a chart 900 illustrating example pretreatment subscores stored in a database. **Figure 10** depicts a chart 1000 illustrating example extraction subscores stored in a database. **Figure 11** depicts a chart 1100 illustrating example post processing subscores stored in a database. **Figure 12** depicts a chart 1200 illustrating example total scores stored in a database. In some aspects, any or all of the charts 900-1200 may display as a visualization of the quantitative brine analysis.

Thus, a visualization can be provided with indicators corresponding to the feasibility of assessed brine locations for mineral extraction, where the feasibility accounts for the quantitative brine score based on the geochemistry database.

### *Example System for Quantitative Brine Analysis for Mineral Extraction*

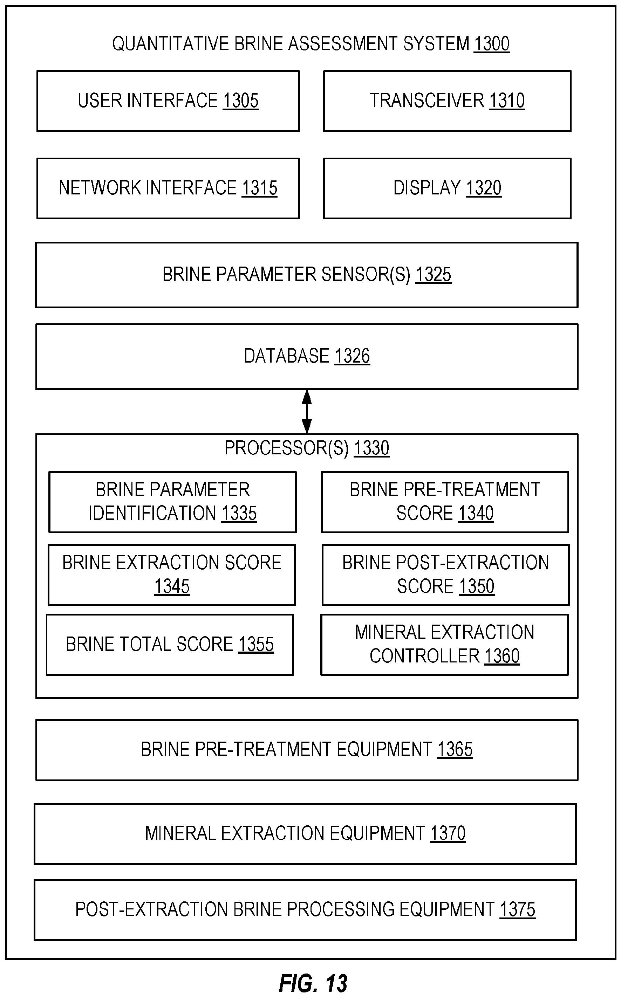

**Figure 13** depicts an example quantitative brine analysis system 1300.

As shown, the quantitative brine analysis system 1300 may include a user interface 1305. In some aspects, the user interface 1305 includes a graphical user interface (GUI) that displays to a user and accepts inputs from the user. In some aspects, the user interface 1305 includes one or more input/output (IOs) interfaces that allows one or more I/O devices (e.g., keyboards, displays, mouse devices, pen inputs, microphones, etc.) to connect to the quantitative brine analysis system 1300. In some aspects, the user interface 1305 may allow a user to input information of the parameters of a brine to the quantitative brine analysis system 1300. In some aspects, the user interface 1305 may allow the user to input one or more selections or commands to the quantitative brine analysis system 1300 related to a mineral extraction process.

As shown, the quantitative brine analysis system 1300 may include a transceiver 1310 and a network interface 1315. In some aspects, the transceiver 1310 and network interface 1315 may allow the quantitative brine analysis system 1300 to connect to a network to obtain information of the parameters of a brine for the quantitative brine analysis system 1300. For example, the transceiver 1310 and network interface 1315 may receive the information of the parameters of the brine from a database or from the brine parameter sensor(s) 1325.

As shown, the quantitative brine analysis system 1300 may include a display 1320. The display 1320 may be configured to display one or more visualizations of brine assessment scores in accordance with techniques described herein

As shown, the quantitative brine analysis system 1300 may include one or more brine parameters sensor(s) 1325. The brine parameters sensor(s) 1325 may include sensors configured to perform any of the brine parameters sensing and/or measurements described herein. In some aspects, the brine parameters sensor(s) 1325 may provide information of the one or more parameters of the brine to the processor(s) 1330 of the quantitative brine analysis system 1300 for use in the quantitative brine assessment.

As shown, the quantitative brine analysis system 1300 may include a processing system including one or more processor(s) 1330. The one or more processor(s) 1330 may comprise one or more central processing units (CPUs). The processing system may further include memory and/or storage, which may be local to the quantitative brine analysis system 1300 or remote (e.g., cloud storage). The CPU may retrieve and execute programming instructions stored in the memory. Similarly, the CPU may retrieve and store application data residing in the memory. The CPU may have multiple processing cores. The memory may represent a random access memory (RAM). The storage may be a disk drive, a combination of fixed or removable storage devices, such as fixed disc drives, removable memory cards or optical storage, network attached storage (NAS), or a storage area-network (SAN).

As shown, the one or more processor(s) 1330 may include a brine parameter identification processor 1330, a brine pre-treatment score processor 1340, a brine extraction score processor 1345, a brine post-extraction score processor 1350, a brine total score processor 1355, and a mineral extraction controller 1360.

The brine parameter identification processor 1330 may be configured to identify parameters of a brine in accordance with techniques described herein. For example, the parameters may be input from a user via the user interface 1305, retrieved from a database via the transceiver 1310 and network interface 1315, and/or received from one or more sensors 1325.

The brine pre-treatment score processor 1340 may be configured to determine a brine pre-treatment score in accordance with techniques described herein.

The brine extraction score processor 1345 may be configured to determine a brine extraction score in accordance with techniques described herein.

The brine post-extraction score processor 1350 may be configured to determine a brine post-extraction score in accordance with techniques described herein.

The brine total score processor 1355 may be configured to determine a brine total score in accordance with techniques described herein.

The mineral extraction controller 1360 may be configured to control mineral extraction process in accordance with techniques described herein. In some aspects, the mineral extraction controller 1360 may be configured to control brine pre-treatment equipment 1365, mineral extraction equipment 1370, and post-extraction brine processing equipment 1375 based on the brine assessment scores in accordance with techniques described herein.

As shown the quantitative brine assessment system 1300 may include a database 1326. In some aspects, the database 1326 may store scores generated by the processor(s) 1330. In some aspects, the database 1326 may store the information about the brine composition.

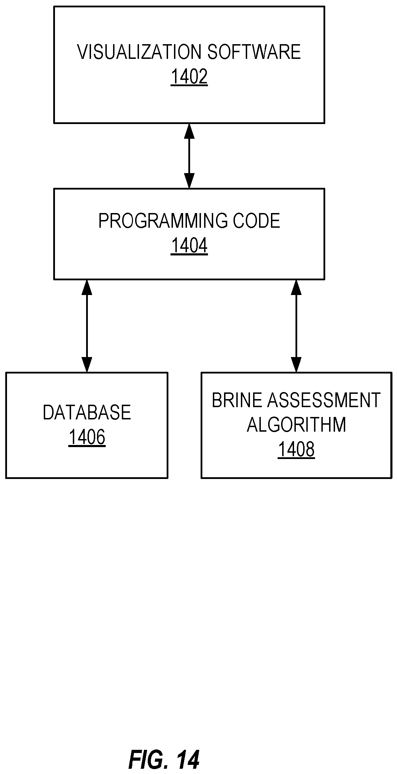

**Figure 14** illustrates example code for brine assessment, brine assessment visualization, and assessing a brine database. As shown, the programming code 1402 may control the visualization software 1404, the brine assessment algorithm 1406, and may link to the database 1408.

*Example Aspects*

Implementation examples are described in the following numbered clauses:

Clause 1: A method for assessing the quality of brines for mineral extraction comprising: obtaining parameters of a sample brine extracted from an aqueous source; determining a brine pretreatment score based on one or more of the parameters of the sample brine; determining a brine extraction score based on one or more of the parameters of the sample brine; determining a brine post-extraction score based on one or more of the parameters of the sample brine; determining a brine total score based on the brine pretreatment score, the brine extraction score, and the brine post-extraction score; and outputting a visualization of at least one of: the brine pretreatment score, the brine extraction score, the brine post-extraction score, or the brine total score to a display.

Clause 2: The method of Clause 1, wherein the obtaining the parameters of the sample brine extracted from the aqueous source comprises: measuring, via one or more sensors, the parameters of the sample brine; receiving an input indicating the measured parameters of the sample brine.

Clause 3: The method of any combination of Clauses 1-2, wherein the parameters of the sample brine comprise a plurality of parameters, i, including a plurality of: a temperature of the sample brine, a total dissolved solids (TDS) of the sample brine, a pH level of the brine, a concentration of one or more components present in the sample brine, or a combination thereof.

Clause 4: The method of any combination of Clauses 1-3, wherein the determining the brine pretreatment score, the brine extraction score, and the brine post-extraction score comprises, for each respective score: for each parameter, $i$, of a total number of the parameters, n, applying a respective coefficient value based on whether or not the parameter is harmful to the respective pre-treatment, extraction, or post-extraction processes; applying a respective weight value to each of the respective coefficient values based on an impact or cost associated with the respective parameter for the respective process; applying a respective function to each of the parameters based on the value of that parameter in the brine; and determining the respective score based on a the calculated values of the respective function over all of the parameters

Clause 5: The method of Clause 4, wherein: the respective coefficient values is 0 if the parameter $i$ as measured in the brine sample does not influence the respective process or 1 if the parameter $i$ as measured in the brine sample influences the respective process; and the respective weight value is based on at least one of: a cost of a brine treatment stage (for instance, to change the value of a parameter so that it the sorbent material used for a mineral extraction process), a process efficiency, a capital cost, an operating cost, a technical feasibility, an environmental impact, an environment regulation, a different regulation, or a combination therefore, associated with the parameter.

Clause 6: The method of any combination of Clauses 4-5, wherein the value of the respective coefficient for each parameter $i$ is determined based on a comparison of a value of the parameter $i$ to at least a threshold.

Clause 7: The method of any combination of Clauses 4-6, further comprising determining the respective function for each of the parameters based on the quantity of the parameter associated with the brine sample and a plurality of empirical coefficients associated with the parameter.

Clause 8: The method of any combination of Clauses 4-7, wherein the determining the brine pretreatment score based on the one or more parameters of the sample brine comprises applying the respective coefficient values based on whether or not the parameter is harmful to a sorbent material used for a mineral extraction process for a brine associated with the parameter.

Clause 9: The method of Clause 8, wherein the determining the brine pretreatment score comprises computing the brine pretreatment score as:

$$PTS = 100 \text{ X } (1 - \frac{\sum_{i=1}^{n} S_i W_i P_i}{\sum_{i=1}^{n} W_i}),$$

wherein $S_i$ is the respective coefficient value, $W_i$ is the respective weight value, and $P_i$ is the respective function applies to the parameters.

Clause 10: The method of any combination of Clauses 4-5, wherein the determining the brine extraction score based on the one or more parameters of the sample brine comprises applying the respective coefficient value based on whether or not the parameter affects the mineral extraction process for a brine associated with the parameter;

Clause 11: The method of Clause 10, wherein the determining the brine extraction score comprises computing the extraction score as:

$$ES = 100 \text{ X } (\frac{\sum_{i=1}^{n} S_i' W_i' P_i'}{\sum_{i=1}^{n} W_i'}),$$

wherein $S_i'$ is the respective coefficient value, $W_i'$ is the respective weight value, and wherein $P_i'$ is the respective function applied to the parameters.

Clause 12: The method of any combination of Clauses 4-5, wherein the determining the brine post-extraction score based on the one or more parameters of the sample brine comprises applying the respective coefficient value based on whether or not the parameter affects the post-extraction stages for a brine associated with the parameter.

Clause 13: The method of Clause 12, wherein the determining the brine post-extraction score comprises computing the brine post-extraction score as:

$$PES = 100 \text{ X } (1 - \frac{\sum_{i-1}^{n} S_i'' W_i'' P_i''}{\sum_{i=1}^{n} W_i''}),$$

wherein $S_i''$ is the respective coefficient value, $W_i''$ is the respective weight value, and wherein $P_i''$ is the respective function applied to the parameters.

Clause 14: The method of any combination of Clauses 1-13, wherein the determining the brine total score comprises: applying a first weight value to the brine pre-treatment score; applying a second weight value to the brine extraction score; applying a third weight value to the brine post-extraction score; and determining the brine total score as a weighted average of the brine pre-treatment subscore, the brine extraction subscore, and the brine post-extraction subscore.

Clause 15: The method of any combination of Clauses 1-14, wherein the visualization comprises a geographic map overlaid with indicators, or one or more charts organized based on different filters such as country, state, basin, region, formation, and reserve, of respective brine pretreatment scores, brine extraction scores, brine post-extraction scores, or brine total scores at a plurality of respective geographic locations of a plurality of brines on the geographic map.

Clause 16: The method of any combination of Clauses 1-15, wherein the visualization comprises a geographic map overlaid with indicators, or one or more charts organized based on different filters such as country, state, basin, region, formation, and reserve, of respective brine feasibility for mineral extraction at a plurality of respective geographic locations of a plurality of brines on the geographic map.

Clause 17: The method of any combination of Clauses 1-16, further comprising selecting the brine for performing the mineral extraction based on the total score.

Clause 18: The method of any combination of Clauses 1-17, further comprising selecting one or more brine pre-treatments to perform before the mineral extraction process based on the brine pre-treatment score.

Clause 19: The method of any combination of Clauses 1-18, further comprising selecting one or more brine post-processing treatments to perform after, or as part of, the mineral extraction process based on the brine post-processing score.

Clause 20: An apparatus, comprising: a memory comprising executable instructions; and one or more processors configured to execute the executable instructions and cause the apparatus to perform a method in accordance with any one of Clauses 1-19.

Clause 21: An apparatus, comprising means for performing a method in accordance with any one of Clauses 1-19.

Clause 22: A non-transitory computer-readable medium comprising executable instructions that, when executed by one or more processors of an apparatus, cause the apparatus to perform a method in accordance with any one of Clauses 1-19.

Clause 23: A computer program product embodied on a computer-readable storage medium comprising code for performing a method in accordance with any one of Clauses 1-19.

Clause 24: A system for performing a method in accordance with any one of Clauses 1-19.

*Additional Considerations*

The preceding description is provided to enable any person skilled in the art to practice the various aspects described herein. The examples discussed herein are not limiting of the scope, applicability, or aspects set forth in the claims. Various modifications to these aspects will be readily apparent to those skilled in the art, and the general principles defined herein may be applied to other aspects. For example, changes may be made in the function and arrangement of aspects discussed without departing from the scope of the disclosure. Various examples may omit, substitute, or add various procedures or aspects as appropriate. For instance, the methods described may be performed in an order different from that described, and various actions may be added, omitted, or combined. Also, features described with respect to some examples may be combined in some other examples. For example, an apparatus may be implemented or a method may be practiced using any number of the aspects set forth herein. In addition, the scope of the disclosure is intended to cover such an apparatus or method that is practiced using other structure, functionality, or structure and functionality in addition to, or other than, the various aspects of the disclosure set forth herein. It should be understood that any aspect of the disclosure disclosed herein may be embodied by one or more elements of a claim.

The various illustrative logical blocks, modules and circuits described in connection with the present disclosure may be implemented or performed with a general purpose processor, a digital signal processor (DSP), an ASIC, a field programmable gate array (FPGA) or other programmable logic device (PLD), discrete gate or transistor logic, discrete hardware components, or any combination thereof designed to perform the functions described herein. A general-purpose processor may be a microprocessor, but in the alternative, the processor may be any commercially available processor, controller, microcontroller, or state machine. A processor may also be implemented as a combination of computing devices, e.g., a combination of a DSP and a microprocessor, a plurality of microprocessors, one or more microprocessors in conjunction with a DSP core, a system on a chip (SoC), or any other such configuration.

As used herein, a phrase referring to "at least one of" a list of items refers to any combination of those items, including single members. As an example, "at least one of: a, b, or c" is intended to cover a, b, c, a-b, a-c, b-c, and a-b-c, as well as any combination with multiples of the same element (e.g., a-a, a-a-a, a-a-b, a-a-c, a-b-b, a-c-c, b-b, b-b-b, b-b-c, c-c, and c-c-c or any other ordering of a, b, and c).

As used herein, the term "determining" encompasses a wide variety of actions. For example, "determining" may include calculating, computing, processing, deriving, investigating, looking up (e.g., looking up in a table, a database or another data structure), ascertaining and the like. Also, "determining" may include receiving (e.g., receiving information), accessing (e.g., accessing data in a memory) and the like. Also, "determining" may include resolving, selecting, choosing, establishing and the like.

The methods disclosed herein comprise one or more actions for achieving the methods. The method actions may be interchanged with one another without departing from the scope of the claims. In other words, unless a specific order of actions is specified, the order and/or use of specific actions may be modified without departing from the scope of the claims. Further, the various operations of methods described above may be performed by any suitable means capable of performing the corresponding functions. The means may include various hardware and/or software component(s) and/or module(s), including, but not limited to a circuit, an application specific integrated circuit (ASIC), or processor.

The following claims are not intended to be limited to the aspects shown herein, but are to be accorded the full scope consistent with the language of the claims. Within a claim, reference to an element in the singular is not intended to mean "one and only one" unless specifically so stated, but rather "one or more." Unless specifically stated otherwise, the term "some" refers to one or more. No claim element is to be construed under the provisions of 35 U.S.C. §112(f) unless the element is expressly recited using the phrase "means for". All structural and functional equivalents to the elements of the various aspects described throughout this disclosure that are known or later come to be known to those of ordinary skill in the art are expressly incorporated herein by reference and are intended to be encompassed by the claims. Moreover, nothing disclosed herein is intended to be dedicated to the public regardless of whether such disclosure is explicitly recited in the claims.

**Claims**

1. A method for assessing the quality of brines for mineral extraction comprising:

   obtaining parameters of a sample brine extracted from an aqueous source;
   determining a brine pretreatment score based on one or more of the parameters of the sample brine;
   determining a brine extraction score based on one or more of the parameters of the sample brine;
   determining a brine post-extraction score based on one or more of the parameters of the sample brine;
   determining a brine total score based on the brine pretreatment score, the brine extraction score, and the brine post-extraction score; and
   outputting a visualization of at least one of: the brine pretreatment score, the brine extraction score, the brine post-extraction score, or the brine total score to a display.

2. The method of claim 1, wherein the parameters of the sample brine comprise a plurality of parameters, i, including a plurality of: a temperature of the sample brine, a total dissolved solids (TDS) of the sample brine, a pH level of the brine, a concentration of one or more components present in the sample brine, or a combination thereof.

3. The method of any one of the claims 1 or 2, wherein the determining the brine pretreatment score, the brine extraction score, and the brine post-extraction score comprises, for each respective score:

   for each parameter, i, of a total number of the parameters, n, applying a respective coefficient value based on whether or not the parameter influences the respective pre-treatment, extraction, or post-extraction processes;
   applying a respective weight value to each of the respective coefficient values based on an impact or cost associated with the respective parameter for the respective process;

   applying a respective function to each of the parameters based on the value of that parameter in the brine; and
   determining the respective score based on the calculated values of the respective function over all of the parameters

4. The method of claim 3, wherein:

   the respective coefficient values is 0 if the parameter i as measured in the brine sample does not influence the respective process or 1 if the parameter i as measured in the brine sample influences the respective process; and
   the respective weight value is based on at least one of: a cost of a brine treatment stage, a process efficiency, a capital cost, an operating cost, a technical feasibility, an environmental impact, an environment regulation, a different regulation, or a combination therefore, associated with the parameter.

5. The method of any one of the claim 3 or 4, further comprising determining the respective function for each of the parameters based on the quantity of the parameter associated with the brine sample and a plurality of empirical coefficients associated with the parameter.

6. The method of any one of the claims 3 to 5, wherein the determining the brine pretreatment score based on the one or more parameters of the sample brine comprises applying the respective coefficient values based on whether or not the parameter is harmful to a sorbent material used for a mineral extraction process for a brine associated with the parameter.

7. The method of claim 6, wherein the determining the brine pretreatment score comprises computing the brine pretreatment score as:

$$PTS = 100 \times \left(1 - \frac{\sum_{i=1}^{n} S_i W_i P_i}{\sum_{i=1}^{n} W_i}\right),$$

wherein $S_i$ is the respective coefficient value, $W_i$ is the respective weight value, and $P_i$ is the respective function applies to the parameters.

8. The method of any one of the claims 3 to 7, wherein the determining the brine extraction score based on the one or more parameters of the sample brine comprises applying the respective coefficient value based on whether or not the parameter affects the mineral extraction process for a brine associated with the parameter.

9. The method of claim 8, wherein the determining the brine extraction score comprises computing the extraction score as:

$$ES = 100 \times \left(\frac{\sum_{i=1}^{n} S_i{}' W_i{}' P_i{}'}{\sum_{i=1}^{n} W_i{}'}\right),$$

wherein $S_i{}'$ is the respective coefficient value, $W_i{}'$ is the respective weight value, and wherein $P_i{}'$ is the respective function applied to the parameters.

10. The method of any of the claims 3 to 9, wherein the determining the brine post-extraction score based on the one or more parameters of the sample brine comprises applying the respective coefficient value based on whether or not the parameter affects the post-extraction stages for a brine associated with the parameter.

11. The method of claim 10, wherein the determining the brine post-extraction score comprises computing the brine post-extraction score as:

$$PES = 100 \times \left(1 - \frac{\sum_{i=1}^{n} S_i{}'' W_i{}'' P_i{}''}{\sum_{i=1}^{n} W_i{}''}\right),$$

wherein $S_i{}''$ is the respective coefficient value, $W_i{}''$ is the respective weight value, and wherein $P_i{}''$ is the respective function applied to the parameters.

12. The method of any one of the claims 1 to 11, wherein the determining the brine total score comprises:

applying a first weight value to the brine pre-treatment score;
applying a second weight value to the brine extraction score;
applying a third weight value to the brine post-extraction score; and
determining the brine total score as a weighted average of the brine pre-treatment subscore, the brine extraction subscore, and the brine post-extraction subscore.

13. The method of any one of the claims 1 to 12, wherein the visualization comprises a geographic map overlaid with indicators, or one or more charts organized based on different filters such as country, state, basin, region, formation, and reserve, of respective brine pretreatment scores, brine extraction scores, brine post-extraction scores, or brine total scores at a plurality of respective geographic locations of a plurality of brines on the geographic map.

14. The method of any of the claims 1 to 13, wherein the visualization comprises a geographic map overlaid with indicators, or one or more charts organized based on different filters such as country, state, basin, region, formation, and reserve, of respective brine feasibility for mineral extraction at a plurality of respective geographic locations of a plurality of brines on the geographic map.

15. A non-transitory computer-readable medium storing computer-executable code for performing a method according to any one of the preceding claims.

**FIG. 1**

FIG. 2

Raw brine — 302

303

L I+ < a —Yes→ Brine processing uneconomical — 304

No

305

L I+ > b —Yes→ Bypass brine pre-treatment — 306

Yes

308

MFR (TSS > 100 µm) > thresh1 —Yes→ Gravity separation — 310

No

312

MFR (Oil > 50 µm) > thresh2 —Yes→ Physical separation — 314

No

316

MFR (TSS > 10 µm) > thresh3 —Yes→ Physical separation, sieve — 318

No

320

MFR (Grease /Oil > 5 µm) > thresh4 —Yes→ Skim tanks, plate pack interceptors, API separator — 322

No

324

TSS > 10 mg/L —Yes→ Hydrocyclone, filtration, centrifuge, electrochemical — 326

No

To FIG. 3B

300

**FIG. 3A**

No

330

Gas flotation, hydrocyclone, centrifuge, filtration, membrane separation

328

Yes

Oil droplets and dispersed hydrocarbons > 5 mg/L

No

334

Gas purging, chemical treatment

332

Yes

Dissolved gases > 50 mg/L

No

338

Adsorption, absorption, membrane separation, filtration, oxidation, bio-treatment

336

Yes

Dissolved hydrocarbons > 5 mg/L or COD > 50 mg/L

No

342

Chemical treatment, bio-treatment

340

Yes

Organics > 10 mg/L

No

346

Chemical disinfection, chemical treatment, filtration, UV radiation

344

Yes

Microorganisms count > 10^4 cfu/ml & TOC > 2 mg/L or BOD > 30 mg/L

No

350

Mebrane separation, activated carbon, chemical treatment, adsorption, absorption

348

Yes

Production chemicals > 5 mg/L

No

**FIG. 3B**

300

*From FIG. 3B*

**354** Gas purging, activated carbon, chemical treatment, adsorption, absorption

No

**352** Dissolved gases > 5 mg/L — Yes

No

**358** Chemical precipitation, nanofiltration, ultrafiltration, membrane separation, biological treatment

**356** Sulfate > 50 mg/L — Yes

No

**362** Chemical treatment, ion exchange, membrane separation

**360** Silica > 5 mg/L — Yes

No

**366** Chemical treatment, chemical precipitation, membrane separation, nanofiltration, ultrafiltration, solvent extraction

**364** Al > 5 mg/L — Yes

No

**370** Chemical treatment

**368** Fe > 5 mg/L or Mn > 50 mg/L — Yes

*To FIG. 3D*

**FIG. 3C**

300

<u>*From FIG. 3C*</u>

No

374

Chemical treatment,
physical separation, reverse
osmosis, ion exchange

372

Yes

Zn
> 25 mg/L

No

378

Precipitation, ion
exchange, reverse
osmosis, coagulation,
filtration

376

Yes

Other heavy metals and
NORMs

No

382

Chemical treatment

380

Yes

pH > 6

No

<u>*Direct extraction of
element of interest*</u>

**FIG. 3D**

300

A METHOD FOR RECOVERY OF AN ELEMENT OF INTEREST

MEASURE ONE OR MORE PROPERTIES OF A SAMPLE BRINE EXTRACTED FROM THE AQUEOUS SOURCE — 405

COMPARE THE MEASUREMENTS OF THE ONE OR MORE PROPERTIES OF THE SAMPLE BRINE TO ONE OR MORE SPECIFIED THRESHOLDS OR RANGES — 410

SELECT ONE OR MORE BRINE PRE-TREATMENT STAGES, OF A PLURALITY OF CONFIGURED BRINE PRE-TREATMENT STAGES, BASED ON THE COMPARISON OF THE ONE OR MORE PROPERTIES OF THE SAMPLE BRINE TO THE ONE OR MORE SPECIFIED THRESHOLDS OR RANGES — 415

PERFORM THE SELECTED ONE OR MORE BRINE PRE-TREATMENT STAGES ON A PROCESS BRINE EXTRACTED FROM THE AQUEOUS SOURCE — 420

BEGIN EXTRACTION — 425

*FIG. 4* — 400

A WORKFLOW FOR QUANTITATIVE BRINE ANALYSIS

IDENTIFY BRINE COMPOSITION — 505

DETERMINE A BRINE PRE-TREATMENT SCORE — 510

DETERMINE A BRINE EXTRACTION SCORE — 515

DETERMINE A BRINE POST EXTRACTION SCORE — 520

DETERMINE A BRINE TOTAL SCORE — 525

SELECT A BRINE FOR PERFORMING A MINERAL EXTRACTION PROCESS BASED ON THE SCORES — 530

SELECT ONE OR MORE BRINE PRE-TREATMENTS FOR THE MINERAL EXTRACTION PROCESS BASED ON THE BRINE PRE-TREATMENT SCORE — 535

SELECT ONE OR MORE BRINE POST-PROCESSING TREATMENTS FOR THE MINERAL EXTRACTION PROCESS BASED ON THE BRINE POST-PROCESSING SCORE — 540

PERFORM MINERAL EXTRACTION ON THE BRINE — 545

*FIG. 5* — 500

| General brine information | | | Ionic Composition (cont'd) | | |
|---|---|---|---|---|---|
| Parameter i | Value | Unit | Parameter i | Value | unit |
| Natural Temp. | | Celsius | Pb | | mg/L |
| TDS | | Mg/L | S | | mg/L |
| Acidity | | pH | Si | | mg/L |
| **Ionic Composition** | | | $SiO_2$ | | mg/L |
| Al | | mg/L | $SO_4^{2-}$ | | mg/L |
| As | | mg/L | Sr | | mg/L |
| B | | mg/L | Ti | | mg/L |
| $B(OH)_3$ | | mg/L | V | | mg/L |
| Ba | | mg/L | Zn | | mg/L |
| Be | | mg/L | Zr | | mg/L |
| Br | | mg/L | Calc. TDS | | mg/L |
| Ca | | mg/L | **Brine Component Type 1** | | |
| Cd | | mg/L | Component 1 | | mg/L |
| Co | | mg/L | Component 2 | | mg/L |
| $CO_3^{2-}$ | | mg/L | Component 3 | | mg/L |
| Cl | | mg/L | **Brine Component Type 2** | | |
| Cr | | mg/L | Component 4 | | mg/L |
| Cu | | mg/L | Component 5 | | mg/L |
| F | | mg/L | Component 6 | | mg/L |
| $Fe_{total}(Fe^{2+})$ | | mg/L | Component 7 | | mg/L |
| $HCO_3^-$ | | mg/L | **Brine Component Type 3** | | |
| K | | mg/L | Component 8 | | mg/L |
| Li | | mg/L | Component 9 | | mg/L |
| Mg | | mg/L | Component 10 | | mg/L |
| Mn | | mg/L | Component 11 | | mg/L |
| Na | | mg/L | Component 12 | | mg/L |
| $Nh_4^+$ | | mg/L | Component 13 | | mg/L |
| Ni | | mg/L | Component 14 | | mg/L |
| $No_3^-$ | | mg/L | Component 15 | | mg/L |
| P | | mg/L | **Brine Component Type 4** | | |
| $Po_4^{3-}$ | | mg/L | Component 16 | | mg/L |

**FIG. 6A**

600

| Brine Component Type 4 (cont'd) | | |
|---|---|---|
| *Parameter i* | *Value* | *unit* |
| Component 17 | | mg/L |
| Component 18 | | mg/L |
| **Brine Component Type 5** | | |
| Component 19 | | cfu/ml |
| Component 20 | | mg/L |
| Component 21 | | mg/L |
| **Brine Component Type 6** | | |
| Component 22 | | mg/L |
| Component 23 | | mg/L |
| Component 24 | | mg/L |
| Component 25 | | mg/L |
| Component 26 | | mg/L |

600

## FIG. 6B

FIG. 7A

FIG. 7B

**Geographic Visualization of Average Pretreatment Score**

○ PTS range 1   ⊘ PTS range 2
⊘ PTS range 3   ⊗ PTS range 4

Geographic Map

800

**Geographic Visualization of Average Extraction Score**

○ ES range 1   ⊘ ES range 2
⊘ ES range 3   ⊗ ES range 4

Geographic Map

820

**Geographic Visualization of Average Post-processing Score**

○ PPS range 1   ⊘ PPS range 2
⊘ PPS range 3   ⊗ PPS range 4

Geographic Map

840

**Geographic Visualization of Average Total Score**

○ TS range 1   ⊘ TS range 2
⊘ TS range 3   ⊗ TS range 4

Geographic Map

860

*FIG. 8*

*FIG. 9*

FIG. 10

1100

*FIG. 11*

*FIG. 12*

QUANTITATIVE BRINE ASSESSMENT SYSTEM 1300

USER INTERFACE 1305

TRANSCEIVER 1310

NETWORK INTERFACE 1315

DISPLAY 1320

BRINE PARAMETER SENSOR(S) 1325

DATABASE 1326

PROCESSOR(S) 1330

BRINE PARAMETER IDENTIFICATION 1335

BRINE PRE-TREATMENT SCORE 1340

BRINE EXTRACTION SCORE 1345

BRINE POST-EXTRACTION SCORE 1350

BRINE TOTAL SCORE 1355

MINERAL EXTRACTION CONTROLLER 1360

BRINE PRE-TREATMENT EQUIPMENT 1365

MINERAL EXTRACTION EQUIPMENT 1370

POST-EXTRACTION BRINE PROCESSING EQUIPMENT 1375

*FIG. 13*

VISUALIZATION SOFTWARE
1402

PROGRAMMING CODE
1404

DATABASE
1406

BRINE ASSESSMENT
ALGORITHM
1408

*FIG. 14*

| | Europäisches Patentamt European Patent Office Office européen des brevets | **EUROPEAN SEARCH REPORT** | **Application Number** EP 24 21 0602 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2023/009888 A1 (SCHLUMBERGER TECHNOLOGY CORP [US]; SCHLUMBERGER CA LTD [CA] ET AL.) 2 February 2023 (2023-02-02) * paragraphs [0015] - [0036]; figures * | 1-12,15 | INV. C22B3/20 C22B26/12 |
| X | YANG KELI ET AL: "Tracking variations in the abundance and composition of dissolved organic matter in solar ponds of oilfield-produced brine", APPLIED GEOCHEMISTRY, vol. 131, 1 June 2021 (2021-06-01), XP086715037, ISSN: 0883-2927, DOI: 10.1016/J.APGEOCHEM.2021.105008 | 1-12,15 | |
| Y | * paragraphs [2.1.], [2.2.]; table 1 * | 13,14 | |
| Y | DISU BOTELHO ET AL: "Lithium Extraction From North Sea Oilfield Brines Using Ion Exchange Membranes", SPE OFFSHORE EUROPE CONFERENCE & EXHIBITION, ABERDEEN, SCOTLAND, UK, 5 September 2023 (2023-09-05), pages 1-19, XP093236334, DOI: 10.2118/215585-MS * abstract; figure 1 * | 13,14 | TECHNICAL FIELDS SEARCHED (IPC) C22B G01N |

The present search report has been drawn up for all claims

| Place of search | Date of completion of the search | Examiner |
|---|---|---|
| The Hague | 27 February 2025 | Wilhelm-Shalganov, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

             

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 21 0602

This annex lists the patent family members relating to the patent documents cited in the above-mentioned European search report.
The members are as contained in the European Patent Office EDP file on
The European Patent Office is in no way liable for these particulars which are merely given for the purpose of information.

27-02-2025

| Patent document cited in search report | Publication date | Patent family member(s) | | Publication date |
|---|---|---|---|---|
| WO 2023009888 A1 | 02-02-2023 | AR | 126668 A1 | 01-11-2023 |
| | | CA | 3227977 A1 | 02-02-2023 |
| | | CL | 2024000260 A1 | 02-08-2024 |
| | | EP | 4376972 A1 | 05-06-2024 |
| | | WO | 2023009888 A1 | 02-02-2023 |

EPO FORM P0459

For more details about this annex : see Official Journal of the European Patent Office, No. 12/82

# EP 4 549 602 A1

**REFERENCES CITED IN THE DESCRIPTION**

**Patent documents cited in the description**

- US 63595784